# EUROPEAN PATENT SPECIFICATION

(11) **EP 1 575 531 B1**
(45) Date of publication and mention of the grant of the patent: **17.08.2011**
(21) Application number: 03798770.8
(22) Date of filing: 26.09.2003
(51) Int. Cl.: A61K 38/21, A61K 38/00, A61P 25/02

(54) **THERAPIES FOR CHRONIC INFLAMMATORY DEMYELINATING POLYNEUROPATHY USING INTERFERON-BETA**
THERAPIEN FÜR CHRONISCH INFLAMMATORISCHE DEMYELINISIERENDE POLYRADIKUONEUROPATHIE MIT INTERFERON-BETA
THERAPIES POUR LA POLYNEUROPATIE DEMYELINISANTE INFLAMMATOIRE CHRONIQUE UTILISANT L'INTERFERON-BETA

(30) Priority: 27.09.2002 US 414307 P
(43) Date of publication of application: 21.09.2005
(73) Proprietor: Biogen Idec MA Inc., Cambridge, Massachusetts 02142 (US)
(72) Inventor: SANDROCK, Alfred, Newton, MA 02459 (US)
(74) Representative: Adams, Harvey Vaughan John
(86) International application number: PCT/US2003/030532
(87) International publication number: WO 2004/028472

(56) References cited:
- EP-A- 0 154 316
- EP-A- 0 287 075
- EP-A- 0 529 300
- WO-A-00/23114
- WO-A-00/23472
- WO-A-02/36628
- WO-A-95/27502
- WO-A-02/074806
- WO-A2-02/072019
- US-A- 5 019 382
- US-A- 6 127 332
- US-B1- 6 531 122
- CHOUDHARY P P ET AL: "Improvement following interferon beta in chronic inflammatory demyelinating polyradiculoneuropathy." JOURNAL OF NEUROLOGY MAR 1995, vol. 242, no. 4, March 1995 (1995-03), pages 252-253, XP009093295 ISSN: 0340-5354
- RUNKEL L ET AL: "Structural and functional differences between glycosylated and non-glycosylated forms of human interferon-beta (IFN-beta)." PHARMACEUTICAL RESEARCH APR 1998, vol. 15, no. 4, April 1998 (1998-04), pages 641-649, XP002271902 ISSN: 0724-8741
- HADDEN R.D.M.: 'Randomized trial of interferon beta-1a in chronic inflammatory demyelinating polyradiculoneuropathy' NEUROLOGY vol. 53, 1999, pages 57 - 61, XP008057191
- VAN DER MEIDE P.H.: 'Discontinuation of treatment with IFN-beta leads to exacerbation of experimental autoimmune encephalomyelitis in Lewis rats.Rapid reversal of the antiproliferative activity of IFN-beta and excessive expansion of autoreactive T cells as disease promoting mechanisms' JOURNAL OF NEUROIMMUNOLOGY vol. 84, 1998, pages 14 - 23, XP002992388
- MARTINA I S ET AL: 'Chronic motor neuropathies: response to interferon-beta1a after failure of conventional therapies.' JOURNAL OF NEUROLOGY, NEUROSURGERY AND PSYCHIATRY PUBMED:10071099 vol. 66, no. 2, February 1999, pages 197 - 201 ISSN: 0022-3050
- HUGHES R A C ET AL: 'Intramuscular interferon beta-1a in chronic inflammatory demyelinating polyradiculoneuropathy' NEUROLOGY LNKD- DOI:10.1212/WNL.0B013E3181D1A862 vol. 74, no. 8, February 2010, pages 651 - 657
- KUNTZER T ET AL: 'Interferon-beta1a in chronic inflammatory demyelinating polyneuropathy.' NEUROLOGY PUBMED:10522905 vol. 53, no. 6, 12 October 1999, pages 1364 - 1365 ISSN: 0028-3878
- DATABASE EMBASE [Online] ELSEVIER SCIENCE PUBLISHERS, AMSTERDAM, NL 1997 ALAM J ET AL: 'Pharmacokinetics and pharmacodynamics of interferon beta-1a (IFN[beta]-1a) in healthy volunteers after intravenous, subcutaneous or intramuscular administration' Database accession no. EMB-1997243108 & CLINICAL DRUG INVESTIGATION 1997 NZ, vol. 14, no. 1, 1997, pages 35-43, ISSN: 1173-2563
- Biogen, Inc.: "AVONEX Information", , 4 December 2000 (2000-12-04), Retrieved from the Internet: URL:http://web.archive.org/web/20001204221 600/www.avonex.com/q&a/q&a.content.html [retrieved on 2010-06-29]
- DATABASE MEDLINE [Online] US NATIONAL LIBRARY OF MEDICINE (NLM), BETHESDA, MD, US 01 April 2003 VALLAT J-M ET AL: 'Interferon beta-1a as an investigational treatment for CIDP.' Database accession no. NLM12707419 & NEUROLOGY PUBMED:12707419 vol. 60, no. 8 SUPPL 3, 01 April 2003, pages S23 - S28 ISSN: 1526-632X
- VALLAT J-M ET AL: 'Interferon beta-1a as an investigational treatment for CIDP.', 01 April 2003, NEUROLOGY 1 APR 2003 LNKD- PUBMED:12707419, VOL. 60, NR. 8 SUPPL 3, PAGE(S) S23 - S28, ISSN 1526-632X pages S23 - S28

### SEQUENCE LISTING

<110>
   <120> Therapies for chronic inflammatory demyelinating polyneuropathy using Interferon-β
   <130> BII-002.60
   <160> 4
   <170> Patent In version 3.0
<210> 1.
   <211> 840
   <212> DNA
   <213> homo sapiens
   <220>
   <221> CDS
   <222> (76)..(639)
<400> 1
   acattctaac tgcaaccttt cgaagccttt gctctggcac aacaggtagt aggcgacact      60
   gttcgtgttg tcaac atg acc aac aag tgt ctc ctc caa att gct ctc ctg      111
                    Met Thr Asn Lys Cys Leu Leu Gln Ile Ala Leu Leu
                    1               5                   10
   ttg tgc ttc tcc act aca gct ctt tcc atg agc tac aac ttg ctt gga      159
   Leu Cys Phe Ser Thr Thr Ala Leu Ser Met Ser Tyr Asn Leu Leu Gly
       15                  20                  25
   ttc cta caa aga agc agc aat ttt cag tgt cag aag ctc ctg tgg caa      207
   Phe Leu Gln Arg Ser Ser Asn Phe Gln Cys Gln Lys Leu Leu Trp Gln
   30                  35                  40
   ttg aat ggg agg ctt gaa tac tgc ctc aag gac agg atg aac ttt gac      255
   Leu Asn Gly Arg Leu Glu Tyr Cys Leu Lys Asp Arg Met Asn Phe Asp
   45                  50                  55                  60
   atc cct gag gag att aag cag ctg cag cag ttc cag aag gag gac gcc      303
   Ile Pro Glu Glu Ile Lys Gln Leu Gln Gln Phe Gln Lys Glu Asp Ala
                   65                  70                  75
   gca ttg acc atc tat gag atg ctc cag aac atc ttt gct att ttc aga      351
   Ala Leu Thr Ile Tyr Glu Met Leu Gln Asn Ile Phe Ala Ile Phe Arg
               80                  85                  90
   caa gat tca tct agc act ggc tgg aat gag act att gtt gag aac ctc      399
   Gln Asp Ser Ser Ser Thr Gly Trp Asn Glu Thr Ile Val Glu Asn Leu
           95                  100                 105
   ctg gct aat gtc tat cat cag ata aac cat ctg aag aca gtc ctg gaa      447
   Leu Ala Asn Val Tyr His Gln Ile Asn His Leu Lys Thr Val Leu Glu
       110                 115                 120
   gaa aaa ctg gag aaa gaa gat ttc acc agg gga aaa ctc atg agc agt      495
   Glu Lys Leu Glu Lys Glu Asp Phe Thr Arg Gly Lys Leu Met Ser Ser
   125                 130                 135                 140
   ctg cac ctg aaa aga tat tat ggg agg att ctg cat tac ctg aag gcc      543
   Leu His Leu Lys Arg Tyr Tyr Gly Arg Ile Leu His Tyr Leu Lys Ala
                   145                 150                 155
   aag gag tac agt cac tgt gcc tgg acc ata gtc aga gtg gaa atc cta      591
   Lys Glu Tyr Ser His Cys Ala Trp Thr Ile Val Arg Val Glu Ile Leu
               160                 165                 170
   agg aac ttt tac ttc att aac aga ctt aca ggt tac ctc cga aac tga      639
   Arg Asn Phe Tyr Phe Ile Asn Arg Leu Thr Gly Tyr Leu Arg Asn
           175                 180                 185
   agatctccta gcctgtgcct ctgggactgg acaattgctt caagcattct tcaaccagca      699
   gatgctgttt aagtgactga tggctaatgt actgcatatg aaaggacact agaagatttt      759
   gaaattttta ttaaattatg agttattttt atttatttaa attttatttt ggaaaataaa      819
   ttatttttgg tgcaaaagtc a                                                840
<210> 2
   <211> 187
   <212> PRT
   <213> homo sapiens
<400> 2
   Met Thr Asn Lys Cys Leu Leu Gln Ile Ala Leu Leu Leu Cys Phe Ser
   1               5                   10                  15
   Thr Thr Ala Leu Ser Met Ser Tyr Asn Leu Leu Gly Phe Leu Gln Arg
               20                  25                  30
   Ser Ser Asn Phe Gln Cys Gln Lys Leu Leu Trp Gln Leu Asn Gly Arg
           35                  40                  45
   Leu Glu Tyr Cys Leu Lys Asp Arg Met Asn Phe Asp Ile Pro Glu Glu
       50                  55                  60
   Ile Lys Gln Leu Gln Gln Phe Gln Lys Glu Asp Ala Ala Leu Thr Ile
   65                  70                  75                  80
   Tyr Glu Met Leu Gln Asn Ile Phe Ala Ile Phe Arg Gln Asp Ser Ser
                   85                  90                  95
   Ser Thr Gly Trp Asn Glu Thr Ile Val Glu Asn Leu Leu Ala Asn Val
               100                 105                 110
   Tyr His Gln Ile Asn His Leu Lys Thr Val Leu Glu Glu Lys Leu Glu
           115                 120                 125
   Lys Glu Asp Phe Thr Arg Gly Lys Leu Met Ser Ser Leu His Leu Lys
       130                 135                 140
   Arg Tyr Tyr Gly Arg Ile Leu His Tyr Leu Lys Ala Lys Glu Tyr Ser
   145                 150                 155                 160
   His Cys Ala Trp Thr Ile Val Arg Val Glu Ile Leu Arg Asn Phe Tyr
                   165                 170                 175
   Phe Ile Asn Arg Leu Thr Gly Tyr Leu Arg Asn
               180                 185
<210> 3
   <211> 501
   <212> DNA
   <213> homo sapiens
   <220>
   <221> CDS
   <222> (1)..(501)
<400> 3
   atg agc tac aac ttg ctt gga ttc cta caa aga agc agc aat ttt cag        48
   Met Ser Tyr Asn Leu Leu Gly Phe Leu Gln Arg Ser Ser Asn Phe Gln
   1               5                   10                  15
   tgt cag aag ctc ctg tgg caa ttg aat ggg agg ctt gaa tac tgc ctc        96
   Cys Gln Lys Leu Leu Trp Gln Leu Asn Gly Arg Leu Glu Tyr Cys Leu
               20                  25                  30
   aag gac agg atg aac ttt gac atc cct gag gag att aag cag ctg cag       144
   Lys Asp Arg Met Asn Phe Asp Ile Pro Glu Glu Ile Lys Gln Leu Gln
           35                  40                  45
   cag ttc cag aag gag gac gcc gca ttg acc atc tat gag atg ctc cag       192
   Gln Phe Gln Lys Glu Asp Ala Ala Leu Thr Ile Tyr Glu Met Leu Gln
       50                  55                  60
   aac atc ttt gct att ttc aga caa gat tca tct agc act ggc tgg aat       240
   Asn Ile Phe Ala Ile Phe Arg Gln Asp Ser Ser Ser Thr Gly Trp Asn
   65                  70                  75                  80
   gag act att gtt gag aac ctc ctg gct aat gtc tat cat cag ata aac       288
   Glu Thr Ile Val Glu Asn Leu Leu Ala Asn Val Tyr His Gln Ile Asn
                   85                  90                  95
   cat ctg aag aca gtc ctg gaa gaa aaa ctg gag aaa gaa gat ttc acc       336
   His Leu Lys Thr Val Leu Glu Glu Lys Leu Glu Lys Glu Asp Phe Thr
               100                 105                 110
   agg gga aaa ctc atg agc agt ctg cac ctg aaa aga tat tat ggg agg       384
   Arg Gly Lys Leu Met Ser Ser Leu His Leu Lys Arg Tyr Tyr Gly Arg
           115                 120                 125
   att ctg cat tac ctg aag gcc aag gag tac agt cac tgt gcc tgg acc       432
   Ile Leu His Tyr Leu Lys Ala Lys Glu Tyr Ser His Cys Ala Trp Thr
       130                 135                 140
   ata gtc aga gtg gaa atc cta agg aac ttt tac ttc att aac aga ctt       480
   Ile Val Arg Val Glu Ile Leu Arg Asn Phe Tyr Phe Ile Asn Arg Leu
   145                 150                 155                 160
   aca ggt tac ctc cga aac tga                                            501
   Thr Gly Tyr Leu Arg Asn
                   165
<210> 4
   <211> 166
   <212> PRT
   <213> homo sapiens
<400> 4
   Met Ser Tyr Asn Leu Leu Gly Phe Leu Gln Arg Ser Ser Asn Phe Gln
   1               5                   10                  15
   Cys Gln Lys Leu Leu Trp Gln Leu Asn Gly Arg Leu Glu Tyr Cys Leu
               20                  25                  30
   Lys Asp Arg Met Asn Phe Asp Ile Pro Glu Glu Ile Lys Gln Leu Gln
           35                  40                  45
   Gln Phe Gln Lys Glu Asp Ala Ala Leu Thr Ile Tyr Glu Met Leu Gln
       50                  55                  60
   Asn Ile Phe Ala Ile Phe Arg Gln Asp Ser Ser Ser Thr Gly Trp Asn
   65                  70                  75                  80
   Glu Thr Ile Val Glu Asn Leu Leu Ala Asn Val Tyr His Gln Ile Asn
                   85                  90                  95
   His Leu Lys Thr Val Leu Glu Glu Lys Leu Glu Lys Glu Asp Phe Thr
               100                 105                 110
   Arg Gly Lys Leu Met Ser Ser Leu His Leu Lys Arg Tyr Tyr Gly Arg
           115                 120                 125
   Ile Leu His Tyr Leu Lys Ala Lys Glu Tyr Ser His Cys Ala Trp Thr
       130                 135                 140
   Ile Val Arg Val Glu Ile Leu Arg Asn Phe Tyr Phe Ile Asn Arg Leu
   145                 150                 155                 160
   Thr Gly Tyr Leu Arg Asn
                   165

## Description

### Background of the invention

Chronic inflammatory demyelinating polyneuropathy (CIDP) is a neurological disorder characterized by slowly progressive weakness and sensory dysfunction of the legs and arms. This disease is caused by damage to the myelin sheath of the peripheral nerves. Swelling of nerve roots is also a characteristic of the disease. Although it can occur at any age and in both genders, CIDP is more common in young adults, and in men more so than women. Symptoms include tingling or numbness (beginning in the toes and fingers), weakness of the arms and legs, aching pain in the muscles, loss of deep tendon reflexes (areflexia), fatigue, and abnormal sensations.

CIDP is associated with certain other diseases. For example, it has been found that inflammatory demyelinating neuropathies, e.g., CIDP, are diagnosed in one third of human immunodeficiency virus (HIV)-seropositive patients referred for peripheral nerve diseases. CIDP was also found to occur in subjects afflicted with lupus, paraproteinemia, lymphoma or diabetes.

Untreated, CIDP is characterized by accumulating disability that requires physical and occupational therapy, orthotic devices, and long-term treatment. Close follow-up care with a physician knowledgeable in the field is necessary to adjust treatment.

Current methods of treatment for CIDP include administration of corticosteroids, such as prednisone, which may be prescribed alone or in combination with immunosuppressant drugs. Immunosuppressant drugs may also be given in the absence of a steroid. Plasmapheresis (plasma exchange) and intravenous immunoglobulin (IVIg) therapy are also relatively effective and currently being used. IVIg may be used even as a first-line therapy. Also, physiotherapy may improve muscle strength, function and mobility, and minimize the development of contractures.

The course of CIDP varies widely among individuals. Some may have a bout of CIDP followed by spontaneous recovery, while others may have many bouts with partial recovery in between relapses. The disease is a treatable cause of acquired neuropathy and initiation of early treatment to prevent loss of nerve cells is recommended. However, some individuals are left with some residual numbness or weakness.

Thus, the current methods of treatment of CIDP consist of methods that are harmful (e.g., steroids or immunosuppressants); expensive (e.g., IVIg and plasmapheresis); or inconvenient (e.g., plasmapheresis). Accordingly, it would be highly desirable to have effective therapeutic methods for treating chronic demyelinating neuropathies, e.g., CIDP, which are less toxic, less expensive and more convenient than the current methods.

### Summary of the invention

In one embodiment, the invention provides an IFN-β for use in a method for treating a chronic demyelinating motor neuropathy in a mammal, wherein the IFN-β is to be administered intramuscularly. In a preferred embodiment, the neuropathy is chronic inflammatory demyelinating neuropathy (CIDP.).

The IFN-β may comprise human IFN-β. For example, the IFN-β may comprise a protein that is at least about 95% identical to full length mature human IFN-β having SEQ ID NO: 4. The IFN-β may also comprise full length mature human IFN-β having SEQ ID NO: 4. The IFN-β may also comprise full length mature human IFN-β having SEQ ID NO: 4 fused to a heterologous polypeptide, e.g., the constant domain of a human immunoglobulin molecule. The immunoglobulin molecule may be the heavy chain of an IgG1. The IFN-β may comprise SEQ ID NO: 14. The IFN-β may also comprise a pegylated IFN-β.

The IFN-β, or composition comprising such, may comprise a stabilizing agent, such as a protein or amino acid. For example, the stabilizing agent may be arginine. The IFN-β, or composition comprising such, may have a pH between about 4.0 and 7.2.

The IFN-β may be administered once, twice or three times weekly. In certain embodiments, the IFN-β is administered at about 6 or 12 million international units (MIU). The IFN-β is administered intramuscularly . In a preferred embodiment, the subject is a mammal, and preferably a human.

The invention also provides an IFN-β use in a method for treating a chronic demyelinating motor neuropathy, e.g., CIDP, comprising intramuscularly administering to a subject having the chronic demyelinating motor neuropathy a pharmaceutically effective amount of IFN-β and further administering to the subject an immunosuppressant or subjecting the subject to plasmapheresis. The method may comprise administering to the subject an immunosuppressant selected from the group consisting of a steroid, azothioprine, cyclosporin, cyclophosphamide and mycophenolate.

Also within the scope of the invention is an IFN-β for use in treating CIDP, comprising intramuscularly administering to a subject having a CIDP a pharmaceutically effective amount of IFN-β in combination with a second treatment for CIDP, wherein the second treatment is selected from the group consisting of administration of a steroid; administration of IVIg; administration of an anti-inflammatory drug and plasmapheresis . The IFN-β may be admistered weekly, e.g., a weekly administration of about 6 MIU of an IFN-β.

In yet another embodiment, the invention provides an IFN-β for use in a method for treating CIDP in a subject receiving a first CIDP treatment selected from the group consisting of administration of a steroid; administration of an anti-inflammatory drug; administration of IVIG and plasmapheresis, the improvement comprising administering to the subject, in addition to the first CIDP treatment, a dose of IFN-β in an amount effective to significantly reduce the dose or frequency of the first CIDP treatment, wherein administration of the IFN-β is via an intramuscular route, to provide effective relief from symptoms of CIDP. In a further embodiment the improvement comprises intramuscularly administering to the subject, in addition to the first CIDP treatment, once a week a dose of IFN-β in an amount effective to significantly reduce the dose or frequency of the first CIDP treatment, to provide effective relief from symptoms of CIDP. A subject having CIDP may also be treated by receiving a first CIDP treatment selected from the group consisting of administration of a steroid; administration of an anti-inflammatory drug; and plasmapheresis, the improvement comprising intramuscularly admimstering to the subject, in addition to the first CIDP treatment, a dose of IFN-β in an amount effective to significantly reduce the dose or frequency of the first CIDP treatment, to provide effective relief from symptoms of CIDP.

### Brief description of the figures

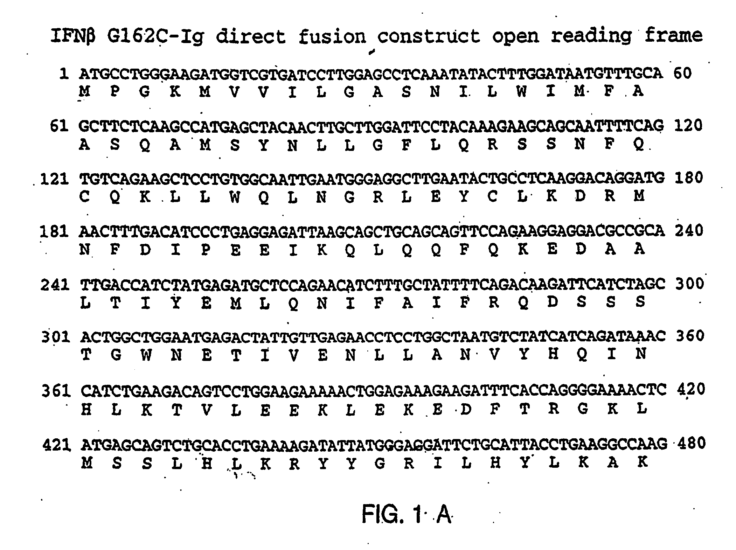

Figs. 1A-C show the nucleotide (SEQ ID NO: 11) and amino acid (SEQ ID NO: 12) sequences of a fusion protein consisting of the VCAM signal sequence fused to the mature full length human IFN-β (SEQ ID NO: 3 and 4), in which the glycine at amino acid 162 of SEQ ID NO: 4 is replaced with a cysteine, fused to the hinge, CH2 and CH3 domains of human IgG1Fc (ZL5107).

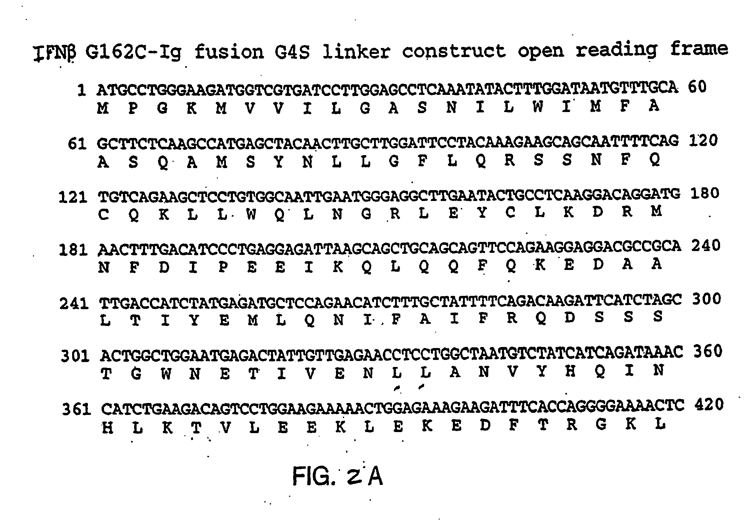

Figs. 2A-C show the nucleotide (SEQ ID NO: 13) and amino acid (SEQ ID NO: 14) sequences of a fusion protein consisting of the VCAM signal sequence fused to the mature full length human human IFN-β (SEQ ID NO: 3 and 4), in which the glycine at amino acid 162 of SEQ ID NO: 4 is replaced with a cysteine; fused to the G4S linker which is fused to the hinge, CH2 and CH3 domains of human IgG1Fc (ZL6206).

### Detailed description of the invention

The invention provides use of an IFN-β in a method for treating chronic demyelinating neuropathies, e.g., CIDP, comprising intramuscularly administering a pharmaceutically effective amount of an IFN-β .

### 1. Definitions:

To more clearly and concisely point out the subject matter of the claimed invention, the following definitions are provided for specific terms used in the written description and the appended claims.

As used in the specification and the appended claims, the singular forms "a," "an" and "the" include plural referents unless the context clearly dictates otherwise.

"Chronic Inflammatory Demyelinating Polyneuropathy" is used interchangeably herein with "CIDP." The following conditions are identical or considered essentially identical to CIDP and are therefore encompassed in the term "CIDP" when used herein: "chronic relapsing polyneuropathy," "chronic idiopathic demyelinating polyneuropathy," "chronic inflammatory demyelinating polyradiculoneuropathy," and "chronic acquired demyelinating polyneuropathy" ("CADP"). CIDP has a chronic progressive, stepwise or relapsing course (see, e.g., Dyck et al. (1993) in Dyck, P.J., Thomas, PIK., Griffin, J.W., Low, P.A., Poduslo, J.F. (Eds), Peripheral Neuropathy, 3rd ed. Saunders, Philadelphia, pp. 1498-1517). The pathological hallmarks of CIDP are segmental demyelination and remyelination and mononuclear cellular infiltrates in the endoneurium (Dyck et al., supra). CIDP is sometimes referred to as the peripheral counterpart of multiple sclerosis (MS) (Toyka and Hartung (1996) Curr. Opin. Neurol. 9, 240-250). It is also sometimes referred to as the chronic form of Guillain-Barre syndrome. Criteria for diagnosis of CIDP include clinical, electrophysiological and cerebrospinal fluid (CSF) criteria, described, e.g., in the report from an adhoc subcommittee of the American Academy of Neurology AIDS taks force (1991) Neurology 41:617. Diagnostic tests include the nine hole peg test; the 10 meters walking test; the Rankin scale or modified form thereof; and an MRC sumscore or modified form thereof (Mathiowetz et al. (1985) Occupational Therapy J. of Res. 5:24; Thompson et al. (1996) J. Neurol. 243:280; Collen et al. (1990) Int. Disability Studies 12:6; van Swieten et al. (1988) Stroke 19:604 and Kleyweg et al. (1991) Muscle Nerve 14:1103). Neurological assessements may also include the Neurologic Disability Scale (NDS); a disability scale (0, healthy; 1, minor signs; 2, able to walk without assistance but unable to run; 3, able to walk 5 meters only with help; 4, chair/bed bound); the Hammersmith Motor Ability Test (HMAT) (Dyck P.J., In "Peripheral Neuropathy" (1993), supra, pages 686-697; Scott et al. (1982) Muscle Nerve 5:291); and testing of nerve conduction. Muscular assessements may involve motor function assessments, e.g., by measuring maximal voluntary isometric contractions (MVIC), as is known in the art; and measurements of muscle strength. Yet other tests of disability include the Ambulation Index; the Functional Independence Measure; Guy's Neurological Disability Scale (GNDS); the Medical Research Council sumscore; the sensory sumscore; and the Hughes functional scale (Hauser et al. (1983) N. Engl. J. Med. 308:173; Hall et al. (1993) J. Head Trauma Rehabilitation 8:60; Sharrack et al. (1996) J. Neurol. 243:S32 and Merkies et al. (2002) Neurology 59:84). Electrophysiological criteria for CIDP were proposed by an Ad Hoc Subcommittee of the American Academy of Neurology (AAN) in 1991, and were recently revised (Nicolas et al. (2002) Muscle Nerve 25:26. Another test that is used for sensory-motor immune-mediated polyneuropathies, e.g., CIDP and Guillain-Barre syndrome (GBS) is the psychometric evaluation of the inflammatory neuropathy cause and treatment (INCAT) sensory sumscore (ISS) (Merkies et al. (2000) Neurology 54:943). Human leukocyte antigens Dw3, DRw3, A1, and B8 occur more frequently in patients with CIDP than in the healthy population (Zvartau-Hind et al. (2002) Chronic Inflammatory Demyelinating Polyradiculoneuropathy, at www.emedicine.com/neuro).

"IFN-β-1a" refers to an IFN-β molecule having the amino acid sequence of the wild-type human IFN-β and is glycosylated.

"IFN-β-1b" refers to an IFN-β molecule having the amino acid sequence of the wild-type IFN-β, wherein the cysteine at position 17 is replaced with a serine; the methione at position 1 ("initiator methionine") is lacking and the molecule is not glycosylated.

"IFN-β variant" refers to a wild-type IFN-β protein having one or more modifications, e.g., amino acid deletions, additions, substitutions, a posttranslational modification or including one or more non-naturally occurring amino acid residues or linkages between them. Portions of IFN-βs are included in the term "IFN-β variant." A "biologically active IFN-β variant" is an IFN-β variant that has at least some activity in treating a neuropathy, e.g., CIDP. An IFN-β variant can be a naturally-occurring IFN-β having, e.g., an insertion, deletion or substitution of one or more amino acids relative to the wild-type IFN-β, i.e., a naturally occurring mutant or a polymorphic variant, or it can be a non-naturally occurring IFN-β.

"International units" or (IU) of IFN-β refers to the units as defined by the World Health Organization (WHO) International Standard for Interferon.

"Isolated" (used interchangeably with "substantially pure") when applied to polypeptides means a polypeptide which, by virtue of its origin or manipulation: (i) is present in a host cell as the expression product of a portion of an expression vector; (ii) is linked to a protein or other chemical moiety other than that to which it is linked in nature; or (iii) does not occur in nature, for example, a protein that is chemically manipulated by appending, or adding at least one hydrophobic moiety to the protein so that the protein is in a form not found in nature. By "isolated" it is further meant a protein that is: (i) synthesized chemically; or (ii) expressed in a host cell and purified away from associated and contaminating proteins. The term generally means a polypeptide that has been separated from other proteins and nucleic acids with which it naturally occurs. Preferably, the polypeptide is also separated from substances such as antibodies or gel matrices (polyacrylamide) which are used to purify it. "Isolated" (used interchangeably with "substantially pure")- when applied to nucleic acids, refers to an RNA or DNA polynucleotide, portion of genomic polynucleotide, cDNA or synthetic polynucleotide which, by virtue of its origin or manipulation: (i) is not associated with all of a polynucleotide with which it is associated in nature (e.g., is present in a host cell as an expression vector or a portion thereof); or (ii) is linked to a nucleic acid or other chemical moiety other than that to which it is linked in nature; or (iii) does not occur in nature. By "isolated" it is further meant a polynucleotide sequence that is: (i) amplified *in vitro* by, for example, polymerase chain reaction (PCR); (ii) synthesized chemically; (iii) produced recombinantly by cloning; or (iv) purified, as by cleavage and gel separation.

"Multifocal motor neuropathy" or "MMN" is a chronic immune mediated demyelinating neuropathy, that is characterized by a stepwise progression of asymmetric muscle weakness and amyotrophy localised in the anatomical distribution areas of peripheral nerves (Pestronk et al. (1988) Ann. Neurol. 24:73 and Komberg et al. (1995) Ann. Neurol. (suppl. 1) S43). The electrohysiological hallmark of multifocal motor neuropathy is persistent conduction block. Clinically, this disease is also described as an asymmetric pure motor variant of CIDP with multifocal motor conduction blocks. During the evolution of multifocal motor neuropathy, the multifocal character may gradually evolve in an essentially symetrical pattern, clinically resembling the motor form of CIDP. Pathological studies have linked these two diseases (Krendel et al. (1996) Ann. Neurol. 40:948 and Oh et al. (1995) Neurology 45:1828). Most patients with multifocal motor neuropathy have high titer antibodies against the ganglioside GM1 (Pestronk et al., supra and Kornberg et al., supra).

A nucleic acid is "operably linked" to another nucleic acid when it is placed into a functional relationship with another nucleic acid sequence. For example, DNA for a presequence or secretory leader (e.g., signal sequence or signal peptide) is operably linked to DNA encoding a polypeptide if the DNA is expressed as a preprotein that participates in the secretion of the polypeptide; a promoter or enhancer is operably linked to a coding sequence if it affects the transcription of the sequence; and a ribosome binding site is operably linked to a coding sequence if it is positioned so as to facilitate translation. Generally, "operably linked" means that the DNA sequences being linked are contiguous and, in the case of, e.g., a secretory leader, contiguous and in reading phase. Linking can be accomplished by ligation, e.g., at convenient restriction sites. If such sites do not exist, synthetic oligonucleotide adaptors or linkers can be used in accordance with conventional practice.

"Percent identity" or "percent similarity" refers to the sequence similarity between two polypeptides, molecules, or between two nucleic acids. When a position in both of the two compared sequences is occupied by the same base or amino acid monomer subunit, then the respective molecules are identical at that position. The percentage identity between two sequences is a function of the number of matching or identical positions shared by the two sequences divided by the number of positions compared x 100. For instance, if 6 of 10 of the positions in two sequences are matched or are identical, then the two sequences are 60% homologous. By way of example, the DNA sequences CTGACT and CAGGTT share 50% homology (3 of the 6 total positions are matched). Generally, a comparison is made when two sequences are aligned to give maximum identity. Such alignment can be provided using, for instance, the method of Karlin and Altschul described in more detail below. When referring to a nucleic acid, "percent homology" and "percent identity" are used interchangeably, whereas when referring to a polypeptide, "percent homology" refers to the degree of similarity, where amino acids representing conserved substitutions of other amino acids are considered identical to these other amino acids. A "conservative substitution" of a residue in a reference sequence is a replacement with an amino acid that is physically or functionally similar to the corresponding reference residue e.g., that have a similar size, shape, electric charge, chemical properties, including the ability to form covalent or hydrogen bonds, or the like. Particularly preferred conservative substitutions are those fulfilling the criteria defined for an "accepted point mutation" in Dayhoff et al., 5: Atlas of Protein Sequence and Structure, 5: Suppl. 3, chapter 22: 354-352, Nat. Biomed. Res. Foundation, Washington, D.C. (1978). The percent homology or identity of two amino acids sequences or two nucleic acid sequences can be determined using the alignment algorithm of Karlin and Altschul (Proc. Nat. Acad. Sci., USA 87: 2264 (1990) as modified in Karlin and Altschul (Proc. Nat Acad. Sci., USA 90: 5873 (1993). Such an algorithm is incorporated into the NBLAST or XBLAST programs of Altschul et al., J. Mol. Biol. 215: 403 (1990). BLAST searches are performed with the NBLAST program, score =100, wordlength =12, to obtain nucleotide sequences homologous to a nucleic acid of the invention. BLAST protein searches are performed with the XBLAST program, score = 50, wordlength = 3, to obtain amino acid sequences homologous to a reference polypeptide. To obtain gapped alignments for comparisons, gapped BLAST is used as described in Altschul et al., Nucleic Acids Res., 25: 3389 (1997). When using BLAST and Gapped BLAST, the default parameters of the respective programs (XBLAST and NBLAST) are used. See http://www/ncbi.nlm.nih.gov.

Quality of life can be measured by the EuroQoL visual analogue scale and the EuroQoL questionnaire sum score; the Medical Outcome Study 36-item short-form health status scale (SF-36); and a Visual Analogue Scale (VAS) (EuroQoL Group (1990) Health Policy 16: 199 and Merkies et al. (2002) Neurology 59:84).

An IFN-β is said to have "therapeutic efficacy," and an amount of the IFN-β is said to be "therapeutically effective," if administration of that amount of the IFN-β alone in combination therapy is sufficient to cause a clinically significant improvement in at least one symptom of a disease relative to the absence of IFN-β treatment. In a preferred embodiment, administration of a therapeutically effective amount of IFN-β in a subject having CIDP results in an improvement of at least one symptom of CIDP, e.g., muscular or neural impairments.

"Wild-type IFN-β" refers to an IFN-β, whether native or recombinant, having the normally occurring amino acid sequence of native IFN-β. The nucleotide and amino acid sequence of native human IFN-β are set forth in SEQ ID NO: 1 and 2, respectively, which are the sequences shown, e.g., in GenBank Accession Nos. M28622 (and E00029) and AAA36040, respectively.

### 2. IFN-β

IFN-β that can be used include wild-type IFN-βs and biologically active variants thereof, e.g., naturally-occurring and non-naturally-occurring variants. The nucleotide and amino acid sequences of wild-type naturally-occurring human IFN-β are set forth in SEQ ID NOs: 1 and 2, respectively, which are identical to GenBank Accession Nos. M28622 and AAA36040, respectively. These IFNs are also described, e.g., in Seghal (1985) J. Interferon Res. 5:521. The full length human IFN-β protein is 187 amino acids long and the coding sequence of SEQ ID NO: 1 corresponds to nucleotides 76-639. The signal sequence corresponds to amino acids 1 to 21. The amino acid sequence of the mature form of this IFN-β corresponds to amino acids 22-187 (nucleotides 139-639 of SEQ ID NO: 1). The mature human IFN-β protein and nucleotide sequence encoding such are set forth as SEQ ID NOs: 4 and 3, respectively.

IFN-β produced in mammalian cells is glycosylated. Naturally-occurring wild-type IFN-β is glycosylated at residue 80 (Asn 80) of the mature polypeptide of SEQ ID NO: 4 or residue 101 (Asn 101) of the immature polypeptide of SEQ ID NO: 2.

IFN-β also includes non-human IFN-βs. e.g., from a vertebrate, such as a mammal, e.g., a non-human primate, bovine, ovine, porcine, equine, feline, canine, rat and mouse; or an avian or amphibian. IFN-β sequences from these species can be obtained from GenBank and/or publications, or can be determined from nucleic acids isolated by low stringency hybridization with an IFN-β gene from another species.

Variants of wild-type IFN-β proteins include proteins having an amino acid sequence that is at least about 70%, 80%, 90%, 95%, 98% or 99% identical or homologous to a wild-type IFN-β, e.g., human IFN-β having SEQ ID NO: 2 or 4. Variants may have one or more amino acid substitutions, deletions or additions. For example, biologically active fragments of wild-type IFN-β proteins can be used. Such fragments may have 1, 2, 3, 5, 10 or up to 20 amino acids deleted, added or substituted at the C- or N-terminus of the protein. Variants may also have 1, 2, 3, 5, 10 or up to 20 amino acid substitutions, deletions or additions. Some variants may have less than about 50, 40, 30, 25, 20, 15, 10, 7, or 5 amino acid substitutions, deletions or additions. Substitutions can be with naturally occurring amino acids or with analogs thereof, e.g., D-stereoisomeric amino acids.

IFN-β variants may be encoded by nucleic acids that hybridize under stringent conditions to a nucleic acid encoding a naturally-occurring IFN-β, e.g., represented by SEQ ID NOs: 1 or 3, or the complement thereof. Appropriate stringency conditions which promote DNA hybridization, for example, 6.0 x sodium chloride/sodium citrate (SSC) at about 45°C, followed by a wash of 2.0 x SSC at 50°C, are known to those skilled in the art or can be found in Current Protocols in Molecular Biology, John Wiley & Sons, N.Y. (1989), 6.3.1-6.3.6; Sambrook et al., 1989, Molecular Cloning, A Laboratory Manual, Cold Spring Harbor Press, N.Y; S. Agrawal (ed.) Methods in Molecular Biology, volume 20; and Tijssen (1993) Laboratory Techniques in biochemistry and molecular biology-hybridization with nucleic acid probes, e.g., part I chapter 2 "Overview of principles of hybridization and the strategy of nucleic acid probe assays", Elsevier, New York. For example, the salt concentration in the wash step can be selected from a low stringency of about 2.0 x SSC at 50°C to a high stringency of about 0.2 x SSC at 50°C. In addition, the temperature in the wash step can be increased from low stringency conditions at room temperature, about 22°C, to high stringency conditions at about 65°C. Both temperature and salt concentration may be varied, or temperature or salt concentration may be held constant while the other variable is changed. Exemplary hybridization conditions include hybridization in 6.0 x sodium chloride/sodium citrate (SSC) at about 50°C, followed by a wash in 0.2 x SSC at room temperature. The temperature of the wash step may be increased to about 45°C, 50°C; 55°C, 60°C, or 65°C to increase the stringency of the hybridization. Hybridization may also be carried out in 5xSSC, 4xSSC, 3xSSC, 2xSSC, 1xSSC or 0.2xSSC. The hybridization can be conducted for at least about 1 hour, 2 hours, 5 hours, 12 hours or 24 hours. The hybridization may also include another agent affecting the stringency, e.g., formamide. For example, stringent hybridization may be conducted in the presence of 50% formamide, which increases the stringency of hybridization at a defined temperature. The wash step may be conducted in the presence of a detergent, e.g., SDS, such as 0.1 or 0.2% SDS. The hybridization may be followed by a wash consisting of a single wash step or at least two wash steps, which may be at the same or a different salinity and temperature. For example, hybridization can be followed by two wash steps at 65°C each for about 20 minutes in 2xSSC, 0.1% SDS, followed by two wash steps at 65°C each for about 20 minutes in 0.2xSSC, 0.1%SDS. Exemplary stringent hybridization conditions include overnight hybridization at 65% in a solution comprising or consisting of 50% formamide, 10xDenhardt (0.2% Ficoll, .2% Polyvinylpyrrolidone, 0.2% bovine serum albumin) and 200 µg/ml of denatured carrier DNA, e.g., sheared salmon sperm DNA, followed by two wash steps at 65°C each for about 20 minutes in 2xSSC, 0.1% SDS, and two wash steps at 65°C each for about 20 minutes in 0.2xSSC, 0.1%SDS. Hybridization may consist of hybridizing two nucleic acids in solution, or a nucleic acid in solution to a nucleic acid attached to a solid support, e.g., a filter. In certain situations, e.g., when one nucleic acid is on a solid support, hybridization may be preceded by a prehybridization step, which may be carried out for at least about 1 hour, 3 hours or 10 hours, and may be in the same solution and at the same temperature as the hybridization solution (without the probe). A nucleic acid encoding an IFN-β variant will hybridize to one of SEQ ID NOs: 1 or 3 or complement thereof under moderately conditions, for example at, and including a wash at, about 2.0 x SSC and about 40°C. A nucleic acid encoding an IFN-β variant will also hybridize to one of SEQ ID NOs: 1 or 3 or complement thereof under high stringency conditions, e.g., at, and including a wash at 0.2 SSC and about 65 °C.

Exemplary modifications are conservative modifications, which have a minimal effect on the secondary and tertiary structure of the protein. Exemplary conservative substitutions include those described by Dayhoff in the Atlas of Protein Sequence and Structure 5 (1978), and by Argos in EMBO J., 8, 779-785 (1989). For example, amino acids belonging to one of the following groups represent conservative changes: ala, pro, gly, gln, asn, ser, thr; cys, ser, tyr, thr; val, ile, leu, met, ala, phe; lys, arg, his; and phe, tyr, trp, his.

Other modifications include the substitution of one amino acid for another amino acid that may not necessarily represent a conservative substitution. For example substitutions that essentially do not affect the three dimensional structure of IFN-β can be made. The three dimensional structure of non-glycosylated human IFN-β is described, e.g., in Radhakrishnan et al. (1996) Structure 4: 1453 and the three dimensional structure of glycosylated IFN-β is described, e.g., in Karpusas et al. (1997) PNAS 94:11813). Essentially, IFN-β comprises five helices: helix A, which consists of about amino acids 2-22 of SEQ ID NO: 4; helix B, which consists of about amino acids 51-71 of SEQ ID NO: 4; helix C, which consists of about amino acids 80-107 of SEQ ID NO: 4; helix D, which consists of about amino acids 118-136 of SEQ ID NO: 4 and helix E, which consists of about amino acids 139-162 of SEQ ID NO: 4 (Karpusas et al., *supra*). Helices A, B, C and E form a left-handed, type 2 four-helix bundle. There is a long overhand loop, the AB loop, that connects helices A and B and three shorter loops (named BC, CD and DE) that connects the rest of the helices (Karpusa et al., *supra).* Previous studies have shown that the N-terminal, C-terminal and the glycosylated C helix regions of the IFN-beta molecule do not lie within the receptor binding site (see, WO 00/23472 and USSN 09/832,659). Accordingly, mutations in these regions would not significantly adversely affect the biological activity of the IFN molecule. It has also been previously shown that mutations in helix C (amino acids 81, 82, 85, 86 and 89 of mature human IFN-β) results in a molecule having higher antiviral activity relative to the wild-type IFN-β (see, WO 00/23472 and USSN 09/832,659). Similarly, it has been shown that mutants in the helix A (amino acids 2, 4, 5, 8 and 11 of mature human IFN-β) and CD loop (amino acids 110, 11,113,116 and 119) have a higher binding activity to the receptor and higher antiviral and anti-proliferative activities relative to the naturally occurring wild-type human IFN-β (see, WO 00/23472 and USSN 09/832,659).

Other preferred modifications or substitutions eliminate sites for intermolecular crosslinking or incorrect disulfide bond formation. For example, IFN-β is known to have three cys residues, at wild-type positions 17, 31 and 141 of SEQ ID NO: 4. One IFN variant is an IFN in which the cys (C) at position 17 has been substituted with ser (S), as described, e.g., in U.S. Pat. No. 4,588,585. Other IFN-β variants include IFN-β variants having, e.g., one or more of ser (S) substituted for cys (C) at position 17 and val (V) at position 101 substituted with phe (F), trp (W), tyr (Y), or his (H), preferably phe (F), when numbered in accordance with wild type IFN-β, having, e.g., SEQ ID NO: 4, such as described, e.g., in U.S. Patent 6,127,332. Other preferred variants include polypeptides having the sequence of a wild-type IFN-β, e.g., having SEQ ID NO: 4, wherein the val (V) at position 101, when numbered in accordance with wild type IFN-β, is substituted with phe (F), tyr (Y), trp (W), his (H), or phe (F), also as described, e.g., in U.S. Patent 6,127,332.

Other IFN-β variants are mature IFN-β molecules lacking the initiator methionine, e.g., methionine 1 of SEQ ID NO: 4. Exemplary IFN-β variants lack an iniatiator methionine and have at least one amino acid substitution, e.g., at position 17 of the mature form, as disclosed in U.S. patent No. 4,588,585.

IFN-β molecules can also be modified by replacing one or more amino acids with one or more derivatized amino acids, which are natural or nonnatural amino acid in which the normally occurring side chain or end group is modified by chemical reaction. Such modifications include, for example, gamma-carboxylation, beta-carboxylation, pegylation, sulfation, sulfonation, phosphorylation, amidization, esterification, N-acetylation, carbobenzylation, tosylation, and other modifications known in the art.

Other modifications include the use of amino acid analogs or derivatized amino acids wherein a side chain is lengthened or shortened while still providing a carboxyl, amino or other reactive precursor functional group for cyclization, as well as amino acid analogs having variant side chains with appropriate functional groups. For instance, the subject compound can include an amino acid analog such as, for example, cyanoalanine, canavanine, djenkolic acid, norleucine, 3-phosphoserine, homoserine, dihydroxyphenylalanine, 5-hydroxytryptophan, 1-methylhistidine, 3-methylhistidine, diaminopimelic acid, ornithine, or diaminobutyric acid. Other naturally occurring amino acid metabolites or precursors having side chains which are suitable herein will be recognized by those skilled in the art.

Other IFN-β variants include reversed or retro peptide sequences. A "reversed" or "retro" peptide sequence refers to that part of an overall sequence of covalently-bonded amino acid residues (or analogs or mimetics thereof) wherein the normal carboxyl-to amino direction of peptide bond formation in the amino acid backbone has been reversed such that, reading in the conventional left-to-right direction, the amino portion of the peptide bond precedes (rather than follows) the carbonyl portion. See, generally, Goodman, M. and Chorev, M. Accounts of Chem. Res. 1979, 12, 423. The reversed orientation peptides described herein include (a) those wherein one or more amino-terminal residues are converted to a reversed ("rev") orientation (thus yielding a second "carboxyl terminus" at the left-most portion of the molecule), and (b) those wherein one or more carboxyl-terminal residues are converted to a reversed ("rev") orientation (yielding a second "amino terminus" at the right-most portion of the molecule). A peptide (amide) bond cannot be formed at the interface between a normal orientation residue and a reverse orientation residue. Therefore, certain reversed polypeptides can be formed by utilizing an appropriate amino acid mimetic moiety to link the two adjacent portions of the sequences utilizing a reversed peptide (reversed amide) bond. In case (a) above, a central residue of a diketo compound may conveniently be utilized to link structures with two amide bonds to achieve a peptidomimetic structure. In case (b) above, a central residue of a diamino compound will likewise be useful to link structures with two amide bonds to form a peptidomimetic structure. The reversed direction of bonding in such polypeptides will generally, in addition, require inversion of the enantiomeric configuration of the reversed amino acid residues in order to maintain a spatial orientation of side chains that is similar to that of the non-reversed peptide. The configuration of amino acids in the reversed portion of the peptides is preferably (D), and the configuration of the non-reversed portion is preferably (L). Opposite or mixed configurations are acceptable when appropriate to optimize a binding activity. Modifications of polypeptides are further described, e.g., in U.S. Patent No. 6,399,075.

IFN-β may be fused to one or more heterologous polypeptides. A heterologous polyeptide may be added, e.g., for the purpose of prolonging the half-life of the IFN-β protein or improving its production. Exemplary heterologous polypeptides include immunoglobulin (Ig) molecules or portions thereof, e.g., the constant domain of a light or heavy chain of an Ig molecule. An IFN-β protein or variant thereof may be fused or otherwise linked to all or part of the hinge and constant regions of an immunoglobulin light chain, heavy chain, or both. Thus, a molecule may include: (1) an IFN-β protein moiety (i.e., an IFN-β or variant thereof), (2) a second peptide, e.g., one which increases solubility or *in vivo* life time of the IFN-β moiety, e.g., a member of the immunoglobulin super family or fragment or portion thereof, e.g., a portion or a fragment of IgG, e.g., the human IgG1 heavy chain constant region, e.g., CH2, CH3, and hinge regions. Specifically, an "IFN-β/Ig fusion" is a protein comprising a biologically active IFN-β moiety linked to the N-terminus of an immunoglobulin chain. A species of IFN-β/Ig fusion is an "IFN-β /Fc fusion" which is a protein comprising an IFN-β moiety linked to at least a portion of the constant domain of an immunoglobulin. A preferred Fc fusion comprises an IFN-β moiety linked to a fragment of an antibody containing the C terminal domain of the heavy immunoglobulin chains.

A fusion protein may comprise an IFN-β polypeptide or variant thereof to which heterologous polypeptides are linked to both its N- and its C-termini. A heterologous polypeptide can also be internal to the IFN-beta polypeptide of variant thereof.

A fusion protein may have the generic formula X-Y-Z, wherein X is a polypeptide having an amino acid sequence of IFN-β, or portion or variant thereof; Y is an optional linker moiety; and Z is a polypeptide comprising at least a portion of a polypeptide other than the interferon beta of moiety X. The fusion protein, may also have the formula Z-Y-X, in which the non-IFN-β polypeptide is fused to the N-terminal portion of the linker which is fused to the N-terminal portion of the IFN-β polypeptide or portion or variant thereof. Moiety Z can be a portion of a polypeptide that contains immunoglobulin-like domains. Examples of such other polypeptides include CD1, CD2, CD4, and members of class I and class II major histocompatability antigens. See U.S. 5,565,335 (Capon et al.) for examples of such polypeptides.

Moiety Z can include, for instance, a plurality of histidine residues or, preferably, the Fc region of an immunoglobulin, "Fc" defined herein as a fragment of an antibody containing the C terminal domain of a heavy immunoglobulin chain.

Moiety Y can be any linker that permits the IFN-β moiety to retain its biological activity. Moiety Y can be one amino acid long or at least two amino acids long. Y can also be from about 2 to about 5 amino acids; from about 3 to about 10 amino acid long or 10 or more amino acids. Preferably Y consists of or comprises GlyGlyGlyGlySer (SEQ ID NO: 6), which is encoded, e.g., by the nucleotide sequence GGCGGTGGTGGCAGC (SEQ ID NO: 5). Y can also consist of or comprise an enterokinase recognition site, e.g., AspAspAspAspLys (SEQ ID NO: 8), which is encoded by, e.g., GACGATGATGACAAG (SEQ ID NO: 7). , Y may also consist of or comprises SerSerGlyAspAspAspAspLys (SEQ ID NO: 10), which is encoded, e.g., by AGCTCCGGAGACGATGATGACAAG (SEQ ID NO: 9).

Moreover, the coupling between the IFN-β moiety (X) and the second, non-IFN-β moiety Z (e.g., an Fc region of an immunoglobulin) can also be effected by any chemical reaction that will bind the two molecules together so long as the X and Z moieties essentially retain their respective activities. This chemical linkage can include many chemical mechanisms such as covalent binding, affinity binding, intercalation, coordinate binding and complexation. Representative coupling agents (i.e., linkers "Y" in the generic formula) to develop covalent binding between the IFN-β moiety and Z moiety can include organic compounds such as thioesters, carbodiimides, succinimide esters, diisocyanates such as tolylene-2,6-diisocyanate, gluteraldehydes, diazobenzenes and hexamethylene diamines such as bis-(p-diazonium-benzoyl)-ethylenediamine, bifunctional derivatives of imidoesters such as dimethyl adipimidate, and bis-active fluorine compounds such as 1,5-difluoro-2,4-dinitrobenzene. This listing is not intended to be exhaustive of the various classes of chemical coupling agents known in the art. Many of these are commercially available such as N-succinimidyl-3-(2-pyridyldithio) propionate (SPDP), 1-ethyl-3-(3-dimethylamino-propyl) carbodiimide hydrochloride (EDC); 4-succinimidyloxycarbonyl-alpha-methyl-alpha-(2-pyridyl-dithio)-toluene (SMPT: Pierce Chem. Co., Cat. # 21558G).

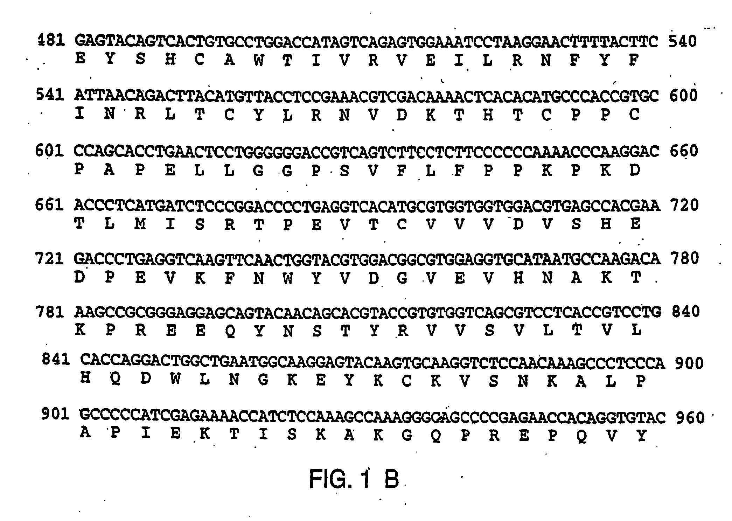

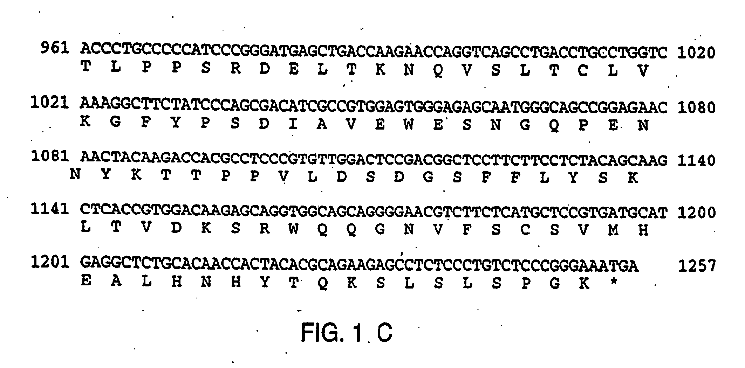

A preferred IFN-β /Ig fusion protein consists of or comprises SEQ ID NO: 12, which contains the full length mature form of human IFN-β, i.e., SEQ ID NO: 4, fused to human IgG1Fc (ZL5107) (see WO 00/23472 and USSN 09/832,659) (see Fig. 1). The corresponding nucleotide sequence is set forth in SEQ ID NO: 11. The DNA encoding human IFN-β ends at nucleotide triplet 568-570 (AAC encoding an arginine) and DNA encoding a human IgG1 constant region starts at the triplet (GAC encoding an aspartic acid) beginning with nucleotide number 574 of SEQ ID NO: 11.

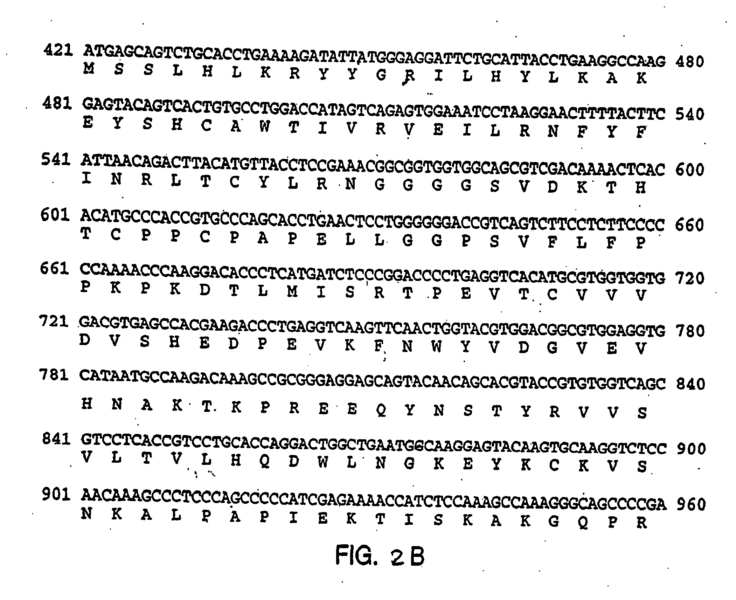

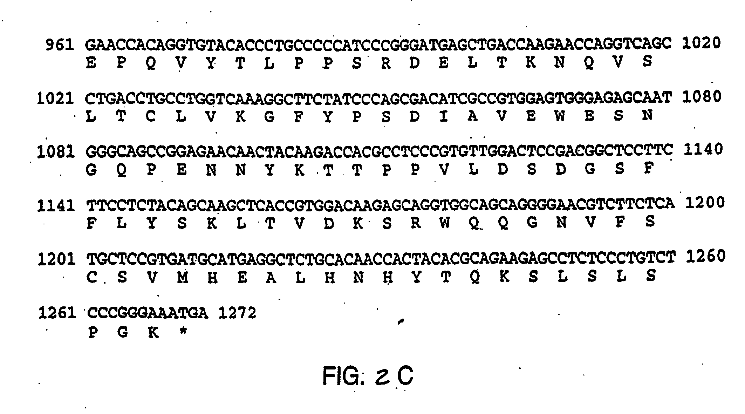

Another preferred IFN-β/Ig fusion protein is set forth in SEQ ID NO: 14 and encoded by SEQ ID NO: 13 (see WO 00/23472 and USSN 09/832,659) (see Fig. 2). This latter fusion protein consists of human IFN-β linked to the G4S linker that is itself linked to human IgG1Fc (ZL6206). The G4S linker (encoded by nucleotides 571 to 585 of SEQ ID NO: 7) consists of the amino acid sequence GGGGS (SEQ ID NO: 9). Methods for producing these proteins are described in WO 00/23472 and USSN 09/832,659.

Preferably, the IFN-β polypeptide is fused via its C-terminus to at least a portion of the Fc region of an immunoglobulin. The IFN-β forms the amino-terminal portion, and the Fc region forms the carboxy terminal portion. In these fusion proteins, the Fc region is preferably limited to the constant domain hinge region and the CH2 and CH3 domains. The Fc region in these fusions can also be limited to a portion of the hinge region, the portion being capable of forming intermolecular disulfide bridges, and the CH2 and CH3 domains, or functional equivalents thereof. These constant regions may be derived from any mammalian source (preferably human) and may be derived from any appropriate class and/or isotype, including IgA, IgD, IgM, IgE and IgG1, IgG2, IgG3 and IgG4.

Recombinant nucleic acid molecules which encode the Ig fusions may be obtained by any method known in the art (Maniatis et al., 1982, Molecular Cloning; A Laboratory Manual, Cold Spring Harbor Laboratory, Cold Spring Harbor, N.Y.) or obtained from publicly available clones. Methods for the preparation of genes which encode the heavy or light chain constant regions of immunoglobulins are taught, for example, by Robinson, R. et al., PCT Application, Publication No. WO87/02671. The cDNA sequence encoding the interferon molecule or fragment may be directly joined to the cDNA encoding the heavy Ig contant regions or may be joined via a linker sequence. A recombinant vector system may be created to accommodate sequences encoding interferon beta in the correct reading frame with a synthetic hinge region. Additionally, it may be desirable to include, as part of the recombinant vector system, nucleic acids corresponding to the 3' flanking region of an immunoglobulin gene including RNA cleavage/polyadenylation sites and downstream sequences. Furthermore, it may be desirable to engineer a signal sequence upstream of the immunoglobulin fusion protein-encoding sequences to facilitate the secretion of the fused molecule from a cell transformed with the recombinant vector.

Dimeric fusion molecules as well as monomeric or multimeric molecules comprising fusion proteins may be provided. Such multimers may be generated by using those Fc regions, or portions thereof, of Ig molecules which are usually multivalent such as IgM pentamers or IgA dimers. It is understood that a J chain polypeptide may be needed to form and stabilize IgM pentamers and IgA dimers. Alternatively, multimers of IFN-β fusion proteins may be formed using a protein with an affinity for the Fc region of Ig molecules, such as Protein A. For instance, a plurality of IFN-β/ immunoglobulin fusion proteins may be bound to Protein A-agarose beads.

These polyvalent forms are useful since they possess multiple interferon beta receptor binding sites. For example, a bivalent soluble IFN-β may consist of two tandem repeats of amino acids 1 to 166 of SEQ ID NO: 4 (or those encoded by nucleic acids numbered 1 to 498 of SEQ. ID. NO: 3) (moiety X in the generic formula) separated by a linker region (moiety Y), the repeats bound to at least a portion of an immunoglobulin constant domain (moiety Z). Alternate polyvalent forms may also be constructed, for example, by chemically coupling IFN-β/Ig fusions to any clinically acceptable carrier molecule, a polymer selected from the group consisting of Ficoll, polyethylene glycol or dextran using conventional coupling techniques. Alternatively, IFN-β may be chemically coupled to biotin, and the biotin-interferon beta Fc conjugate then allowed to bind to avidin, resulting in tetravalent avidin/biotin/interferon beta molecules. IFN-β/Ig fusions may also be covalently coupled to dinitrophenol (DNP) or trinitrophenol (TNP) and the resulting conjugate precipitated with anti-DNP or anti-TNP-IgM, to form decameric conjugates with a valency of 10 for interferon beta receptor binding sites

Derivatives of proteins also include various structural forms of the primary protein which retain biological activity. Due to the presence of ionizable amino and carboxyl groups, for example, IFN-β proteins and variants thereof may be in the form of acidic or basic salts, or may be in neutral form. Individual amino acid residues may also be modified by oxidation or reduction. Further, the primary amino acid structure (including the N- and/or C-terminal ends) or the glycan of the IFN-β may be modified ("derivatized") by forming covalent or aggregative conjugates with other chemical moieties, such as glycosyl groups, polyalkylene glycol polymers such as polyethylene glycol, lipids, phosphate, acetyl groups and the like, or by creating amino acid sequence mutants.

Other derivatives of Interferon beta/ Ig include covalent or aggregative conjugates of interferon beta or its fragments with other proteins or polypeptides, such as by synthesis in recombinant culture as additional N-termini, or C-termini. For example, the conjugated peptide may be a signal (or leader) polypeptide sequence at the N-terminal region of the protein which co-translationally or post-translationally directs transfer of the protein from its site of synthesis to its site of function inside or outside of the cell membrane or wall (e.g., the yeast *alpha* -factor leader). For example, the signal peptide can be that of IFN-β, i.e., amino acids 1-21 of SEQ ID NO: 2, corresponding to nucleotides 76-138 of SEQ ID NO: 1. The signal peptide can also be that of VCAM, i.e., amino acids 1-24 of SEQ ID NO: 12, which is encoded by nucleotides 1-72 ofSEQ ID NO: 11.

A heterologous polypeptide (e.g., peptide) or other molecule may also be used as a label or for helping in the purification of the IFN-β. Such peptides are well known in the art. For example, a polynucleotide may be fused in frame to a marker sequence, also referred to herein as "Tag sequence" encoding a "Tag peptide," which allows for marking and/or purification of the polypeptide . Preferably, the marker sequence is a hexahistidine tag, e.g., supplied by a PQE-9 vector. Numerous other Tag peptides are available commercially. Other frequently used Tags include myc-epitopes (e.g., see Ellison et al. (1991) J Biol Chem 266:21150-21157), which includes a 10-residue sequence from c-myc, the pFLAG system (International Biotechnologies, Inc.), the pEZZ-protein A system (Pharmacia, NJ), and a 16 amino acid portion of the *Haemophilus influenza* hemagglutinin protein. Furthermore, any polypeptide can be used as a Tag so long as a reagent, e.g., an antibody interacting specifically with the Tag polypeptide is available or can be prepared or identified.

An IFN-β protein or variant thereof can be fused at the N- or C-terminus with one of the following peptides: HisHisHis HisHisHis (SEQ ID NO: 16), which may be encoded by the nucleotide sequence CATCATCATCATCATCAT (SEQ ID NO: 15); SerGlyGlyHisHisHisHisHisHis (SEQ ID NO: 18), which may be encoded by the nucleotide sequence TCCGGGGGCCATCATCATCATCATCAT (SEQ ID NO: 15) and SerGlyGlyHisHisHisHisHisHisSerSerGlyAspAspAspAspLys (SEQ ID NO: 20), which may be encoded by the nucleotide sequence TCCGGGGGCCATCATCATCATCATCATAGCTCCGGAGACGATGATGACAAG (SEQ ID NO: 19).

The amino acid sequence of interferon beta can also be linked to the peptide AspTyrLysAspAspAspAspLys (DYKDDDDK) (SEQ ID NO: 21) (Hopp et al., Bio/Technology 6:1204,1988). The latter sequence is highly antigenic and provides an epitope reversibly bound by a specific monoclonal antibody, enabling rapid assay and facile purification of expressed recombinant protein. This sequence is also specifically cleaved by bovine mucosal enterokinase at the residue immediately following the Asp-Lys pairing.

An IFN-β may be fused to an albumin protein, variant or portion thereof. Such a fusion protein can be created as described in, e.g., WO 01/77137.

IFN-β may also include a molecule that is not a polypeptide. For example, an IFN-β protein or variant thereof can be linked covalently or not covalently to a polymer, e.g., a biodegradable polymer. For example, an IFN-β protein or variant thereof can be pegylated, e.g., linked to polyethylene glycol (PEG), as described in WO 00/23114.

A single polymer molecule may be employed for conjugation with an IFN-β, although it is also contemplated that more than one polymer molecule can be attached as well. It will be recognized that the conjugating polymer may utilize any groups, moieties, or other conjugated species, as appropriate to the end use application. By way of example, it may be useful in some applications to covalently bond to the polymer a functional moiety imparting UV-degradation resistance, or antioxidation, or other properties or characteristics to the polymer. As a further example, it may be advantageous in some applications to functionalize the polymer to render it reactive or cross-linkable in character, to enhance various properties or characterisics of the overall conjugated material. Accordingly, the polymer may contain any functionality, repeating groups, linkages, or other constitutent structures which do not preclude the efficacy of the conjugated IFN-β composition for its intended purpose.

The IFN-β is conjugated most preferably via a terminal reactive group on the polymer although conjugations can also be branched from the non-terminal reactive groups. The polymer with the reactive group(s) is designated herein as "activated polymer." The reactive group selectively reacts with free amino or other reactive groups on the protein. The activated polymer(s) are reacted so that attachment may occur at any available IFN-β amino group such as the alpha amino groups or the epsilon-amino groups of lysines. Free carboxylic groups, suitably activated carbonyl groups, hydroxyl, guanidyl, oxidized carbohydrate moieties and mercapto groups of the IFN-β (if available) can also be used as attachment sites.

Although the polymer may be attached anywhere on the IFN-β molecule or variant thereof or other amino acid linked directly or indirectly to the IFN-β molecule, the most preferred site for polymer coupling is the N-terminus of the IFN-β molecule. Secondary site(s) are at or near the C-terminus and through sugar moieties. Thus :(i) N-terminally coupled polymer conjugates of IFN-β or variant thereof; (ii) C-terminally coupled polymer conjugates of IFN-β or variant thereof ; (iii) sugar-coupled conjugates of polymer conjugates; (iv) as well as N-, C- and sugar-coupled polymer conjugates of IFN-β proteins or variants thereof, may be contemplated.

Generally from about 1.0 to about 10 moles of activated polymer per mole of protein, depending on protein concentration, is employed. The final amount is a balance between maximizing the extent of the reaction while minimizing non-specific modifications of the product and, at the same time, defining chemistries that will maintain optimum activity, while at the same time optimizing, if possible, the half-life of the protein. Preferably, at least about 50% of the biological activity of the protein is retained, and most preferably 100% is retained.

The reactions may take place by any suitable method used for reacting biologically active materials with inert polymers, preferably at about pH 5-7 if the reactive groups are on the alpha amino group at the N-terminus. Generally the process involves preparing an activated polymer (that may have at least one terminal hydroxyl group) and thereafter reacting the protein with the activated polymer to produce the soluble protein suitable for formulation. The above modification reaction can be performed by several methods, which may involve one or more steps.

As mentioned above, Preferably utilized are the N-terminal end of IFN-β as the linkage to the polymer. Suitable methods are available to selectively obtain an N-terminally modified IFN-β. One method is exemplified by a reductive alkylation method which exploits differential reactivity of different types of primary amino groups (the epsilon amino groups on the lysine versus the amino groups on the N-terminal methionine) available for derivatization on IFN-β. Under the appropriate selection conditions, substantially selective derivatization of IFN-β at its N-terminus with a carbonyl group containing polymer can be achieved. The reaction is performed at a pH which allows one to take advantage of the pKa differences between the epsilon-amino groups of the lysine residues and that of the alpha-amino group of the N-terminal residue of IFN-β. This type of chemistry is well known to persons with ordinary skill in the art.

For example, a reaction scheme can be used in which this selectivity is maintained by performing reactions at low pH (generally 5-6) under conditions where a PEG-aldehyde polymer is reacted with IFN-β in the presence of sodium cyanoborohydride. After purification of the PEG-IFN-β and analysis with SDS-PAGE, MALDI mass spectrometry and peptide sequencing/mapping, this resulted in an IFN-β whose N-terminus is specifically targeted by the PEG moiety.

The crystal structure of IFN-β indicates that the N- and C-termini are located close to each other (see Karpusas et al., 1997, Proc. Natl. Acad. Sci. 94: 11813-11818). Thus, modifications of the C- terminal end of IFN-β should also have minimal effect on activity. While there is no simple chemical strategy for targeting a polyalkylene glycol polymer such as PEG to the C-terminus, it would be straightforward to genetically engineer a site that can be used to target the polymer moiety. For example, incorporation of a Cys at a site that is at or near the C-terminus would allow specific modification using a maleimide, vinylsulfone or haloacetate- activated polyalkylene glycol (e.g., PEG). These derivatives can be used specifically for modification of the engineered cysteines due to the high selectively of these reagents for Cys. Other strategies such as incorporation of a histidine tag which can be targeted (Fancy et al., (1996) Chem. & Biol. 3: 551) or an additional glycosylation site, represent other alternatives for modifying the C-terminus of IFN-β.

The glycan on the IFN-β is also in a position that would allow further modification without altering activity. Methods for targeting sugars as sites for chemical modification are also well known and therefore it is likely that a polyalkylene glycol polymer can be added directly and specifically to sugars on IFN-β that have been activated trough oxidation. For example, a polyethyleneglycol-hydrazide can be generated which forms relatively stable hydrazone linkages by condensation with aldehydes and ketones. This property has been used for modification of proteins through oxidized oligosaccharide linkages. See Andresz, H. et al., (1978), Makromol. Chem. 179: 301. In particular, treatment of PEG-carboxymethyl hydrazide with nitrite produces PEG-carboxymethyl azide which is an electrophilically active group reactive toward amino groups. This reaction can be used to prepare polyalkylene glycol-modified proteins as well. See, U.S. Patents 4,101,380 and 4,179,337.

Thiol linker-mediated chemistry can further facilitate cross-linking of proteins. This can be performed, e.g., by generating reactive aldehydes on carbohydrate moieties with sodium periodate, forming cystamine conjugates through the aldehydes and inducing cross-linking via the thiol groups on the cystamines (see Pepinsky, B. et al., (1991), J. Biol. Chem., 266: 18244-18249 and Chen, L.L. et al., (1991) J. Biol. Chem., 266: 18237-18243). Accordingly, this type of chemistry is expected to be appropriate for modification with polyalkylene glycol polymers where a linker is incorporated into the sugar and the polyalkylene glycol polymer is attached to the linker. While aminothiol or hydrazine-containing linkers will allow for addition of a single polymer group, the structure of the linker can be varied so that multiple polymers are added and/or that the spatial orientation of the polymer with respect to the IFN-β is changed.

Exemplary polymers include water soluble polymer such as a polyalkylene glycol polymer. A non-limiting list of such polymers include other polyalkylene oxide homopolymers such as polypropylene glycols, polyoxyethylenated polyols, copolymers thereof and block copolymers thereof. Other examples of suitable water-soluble and non-peptidic polymer backbones include poly(oxyethylated polyol), poly(olefinic alcohol), poly(vinylpyrrolidone), poly(hydroxypropylmethacrylamide), poly(α-hydroxy acid), poly(vinyl alcohol), polyphosphazene, polyoxazoline, poly(N-acryloylmorpholine) and copolymers, terpolymers, and mixtures thereof. The polymer backbone may be poly(ethylene glycol) or monomethoxy polyethylene glycol (mPEG) having an average molecular weight from about 200 Da to about 400,000 Da. It should be understood that other related polymers are also suitable for use and that the use of the term PEG or poly(ethylene glycol) is intended to be inclusive and not exclusive in this respect. The term PEG includes poly(ethylene glycol) in any of its forms, including alkoxy PEG, difunctional PEG, multi-armed PEG, forked PEG, branched PEG, pendent PEG, or PEG with degradable linkages therein.

Polyalkylene glycol residues of C1-C4 alkyl polyalkylene glycols, preferably polyethylene glycol (PEG), or poly(oxy)alkylene glycol residues of such glycols are incorporated in the polymer systems of interest. Thus, the polymer to which the protein is attached can be a homopolymer of polyethylene glycol (PEG) or is a polyoxyethylated polyol, provided in all cases that the polymer is soluble in water at room temperature. Non-limiting examples of such polymers include polyalkylene oxide homopolymers such as PEG or polypropylene glycols, polyoxyethylenated glycols, copolymers thereof and block copolymers thereof, provided that the water solubility of the block copolymer is maintained. Examples of polyoxyethylated polyols include, for example, polyoxyethylated glycerol, polyoxyethylated sorbitol, polyoxyethylated glucose, or the like. The glycerol backbone of polyoxyethylated glycerol is the same backbone occurring naturally in, for example, animals and humans in mono-, di-, and triglycerides. Therefore, this branching would not necessarily be seen as a foreign agent in the body.

As an alternative to polyalkylene oxides, dextran, polyvinyl pyrrolidones, polyacrylamides, polyvinyl alcohols, carbohydrate-based polymers and the like may be used. Those of ordinary skill in the art will recognize that the foregoing list is merely illustrative and that all polymer materials having the qualities described herein are contemplated.

The polymer can be of any particular molecular weight, but it is preferred that the molecular weight be between about 300 and 100,000, more preferably between 10,000 and 40,000. In particular, sizes of 20,000 or more are best at preventing protein loss due to filtration in the kidneys.

Polyalkylene glycol derivatization has a number of advantageous properties in the formulation of polymer-IFN-β conjugates, as associated with the following properties of polyalkylene glycol derivatives: improvement of aqueous solubility, while at the same time eliciting no antigenic or immunogenic response; high degrees of biocompatibility; absence of *in vivo* biodegradation of the polyalkylene glycol derivatives; and ease of excretion by living organisms.

Moreover, in another aspect, one can utilize IFN-β covalently bonded to the polymer component in which the nature of the conjugation involves cleavable covalent chemical bonds. This allows for control in terms of the time course over which the polymer may be cleaved from the IFN-β. This covalent bond between the IFN-β drug and the polymer may be cleaved by chemical or enzymatic reaction. The polymer-IFN-β product retains an acceptable amount of activity. Concurrently, portions of polyethylene glycol are present in the conjugating polymer to endow the polymer-IFN-β conjugate with high aqueous solubility and prolonged blood circulation capability. As a result of these improved characteristics parenteral, nasal, and oral delivery of both the active polymer-IFN-β species and, following hydrolytic cleavage, bioavailability of the IFN-β per se, are contemplated in *in vivo* applications.

The reaction of the polymer with the IFN-β to obtain conjugates, e.g., N-terminal conjugated products, can be readily carried out using a wide variety of reaction schemes. The activity and stability of the IFN-β conjugates can be varied in several ways, by using a polymer of different molecular size. Solubilities of the conjugates can be varied by changing the proportion and size of the polyethylene glycol fragment incorporated in the polymer composition.

Conjugates may be prepared by reacting a protein with an activated polyaklylene glycol compound (PCG). For example, IFN can be reacted with a PEG-aldehyde in the presence of a reducing agent (e.g., sodium cyanoborohydride) via reductive alkylation to produce a PEG-protein conjugate, attached via an amine linkage. *See, e.g.,* European Patent 0154316 B1 and International Patent Application No. PCT/US03/01559.

Human IFN-β may be PEGylated with the following activated polyalkylene glycols: 20 kDa mPEG-O-2-methylpropionaldehyde, 20 kDa mPEG-O*-p-*methylphenyl-O-2-methylpropionaldehyde, 20 kDa mPEG-O-*m-*methylphenyl-O-2-methylpropionaldehyde, 20 kDa mPEG-O*-p-*phenylacetaldehyde, 20 kDa mPEG-O*-p-*phenylpropionaldehyde, and 20 kDa mPEG-O*-m-*phenylacetaldehyde to obtain 20 kDa mPEG-O-2-methylpropionaldehyde-modified IFN-β, 20 kDa mPEG-O-*p-*methylphenyl-O-2-methylpropionaldehyde-modified IFN-β, 20 kDa mPEG-O-*m-*methylphenyl-O-2-methylpropionaldehyde-modified IFN-β, 20 kDa mPEG-O-*p-*phenylacetaldehyde-modified IFN-β, 20 kDa mPEG-O-*p*-phenylpropionaldehyde-modified IFN-β, and 20 kDa mPEG-O-*m*-phenylacetaldehyde-modified IFN-β, respectively. A detailed description of the preparation and characterization of human IFN-β modified with 20 kDa mPEG-O-2-methylpropionaldehyde and 20 kDa mPEG-O*-p-*phenylacetaldehyde is set forth below and is also provided in International Patent Application No. PCT/US03/01559.

A pegylated IFN-β is may be prepared as follows. IFN-β, e.g., nonformulated AVONEX^{®} (IFN-β-1a bulk intermediate, (a clinical batch of bulk drug that passed all tests for use in humans), at 250 µg/ml in 100 mM sodium phosphate pH 7.2, 200 mM NaCl) is diluted with an equal volume of 100 mM MES pH 5.0, and the pH was adjusted to 5.0 with HCl. The sample is loaded onto an SP-Sepharose® FF column (Pharmacia, Piscataway, NJ) at 6 mg IFN-β/ml resin. The column is washed with 5 mM sodium phosphate pH 5.5, 75 mM NaCl, and the product is eluted with 30 mM sodium phosphate pH 6.0, 600 mM NaCl. Elution fractions can be analyzed for their absorbance values at 280 nm and the concentration of interferon in the samples estimated from the absorbance using an extinction coefficient of 1.51 for a 1 mg/ml solution.

To a 1 mg/ml solution of the IFN-β from the SP eluate, 0.5 M sodium phosphate pH 6.0 is added to 50 mM, sodium cyanoborohydride (Aldrich, Milwaukee, WI) is added to 5 mM, and 20K PEG aldehyde (Shearwater Polymers, Huntsville, AL) is added to 5 mg/ml. The sample is incubated at room temperature for 20 hours. The pegylated interferon is purified from reaction products by sequential chromatography steps on a Superose® 6 FPLC sizing column (Pharmacia) with 5 mM sodium phosphate pH 5.5, 150 mM NaCl as the mobile phase and SP-Sepharose® FF. The sizing column results in base line separation of modified and unmodified IFN-β. The PEG-interferon beta-containing elution pool from gel filtration is diluted 1:1 with water and loaded at 2 mg interferon beta /ml resin onto an SP-Sepharose® column. The column is washed with 5 mM sodium phosphate pH 5.5, 75 mM NaCl and then the pegylated interferon beta is eluted from the column with 5 mM sodium phosphate pH 5.5, 800 mM NaCl. Elution fractions are analyzed for protein content by absorbance at 280 nm. The pegylated interferon concentration is reported in interferon equivalents as the PEG moiety did not contribute to absorbance at 280 nm. These method and characterization of the pegylated IFN-β obtained are further described in WO 00/23114. PEG conjugation of IFN-β does not appear to alter its antiviral activity. In addition, the specific activity of pegylated IFN-β was found to be much greater (about 10 times) than that of the non-pegylated IFN-β (WO 00/23114).

IFN-β can also be pegylated with a 5K PEG-aldehyde moiety that can be purchased, e.g., from Fluka, Inc. (Cat. No. 75936, Ronkonkoman, NY) following the same protocol as described above for the 20K PEG aldehyde.

A 20 kDa mPEG-O-2-methylpropionaldehyde-modified IFN-β can be prepared as follows. 10 mL of nonformulated AVONEX^{®} (IFN-β-1a bulk intermediate, (a clinical batch of bulk drug that passed all tests for use in humans), at 250 µg/mL in 100 mM sodium phosphate pH 7.2, 200 mM NaCl) is diluted with 12 mL of 165 mM MES pH 5.0 and 50 µL of 5 N HCl. The sample is loaded onto a 300 µL SP-Sepharose FF column (Pharmacia). The column is washed with 3 x 300 µL of 5 mM sodium phosphate pH 5.5, 75 mM NaCl, and the protein is eluted with 5 mM sodium phosphate pH 5.5, 600 mM NaCl. Elution fractions are analyzed for their absorbance at 280 nm and the concentration of IFN-β in the samples estimated using an extinction coefficient of 1.51 for a 1 mg/mL solution. The peak fractions are pooled to give an IFN-β concentration of 3.66 mg/mL, which is subsequently diluted to 1.2 mg/mL with water.

To 0.8 mL of the IFN-β from the diluted SP-Sepharose eluate pool, 0.5 M sodium phosphate pH 6.0 is added to 50 mM, sodium cyanoborohdride (Aldrich) is added to 5 mM, and 20 kDa mPEG-O-2-methylpropionaldehyde is added to 5 mg/mL. The sample is incubated at room temperature for 16 h in the dark. The PEGylated IFN-β is purified from the reaction mixture on a 0.5 mL SP-Sepharose FF column as follows: 0.6 mL of the reaction mixture is diluted with 2.4 mL 20 mM MES pH 5.0, and loaded on to the SP-Sepharose column. The column is washed with sodium phosphate pH 5.5, 75 mM NaCl and then the PEGylated IFN-β is eluted from the column with 25 mM MES pH 6.4, 400 mM NaCl. The PEGylated IFN-β is further purified on a Superose 6 HR 10/30 FPLC sizing column with 5 mM sodium phosphate pH 5.5, 150 mM NaCl as the mobile phase. The sizing column (25 mL) is run at 20 mL/h and 0.5 mL fractions are collected. The elution fractions are analyzed for protein content by absorbance at 280 nm, pooled, and the protein concentration of the pool determined. The PEGylated IFN-β concentration is reported in IFN equivalents as the PEG moiety does not contribute to absorbance at 280 nm. Samples of the pool are removed for analysis, and the remainder can be diluted to 30 µg/mL with HSA-containing formulation buffer, aliquoted at 0.25 mL/vial, and stored at -70 °C.

20 kDa mPEG-O-*p*-phenylacetaldehyde-modified IFN-β can be prepared as follows. 20 mL of nonformulated AVONEX® (IFN-β bulk intermediate, a clinical batch of bulk drug that passed all tests for use in humans, at 250 µg/mL in 100 mM sodium phosphate pH 7.2, 200 mM NaCl) is diluted with 24 mL of 165 mM MES pH 5.0, 100 µL of 5 N HCl, and 24 mL water. The sample is loaded onto a 600 µL SP-Sepharose FF column (Pharmacia). The column is washed with 2 x 900 µL of 5 mM sodium phosphate pH 5.5, 75 mM NaCl, and the protein is eluted with 5 mM sodium phosphate pH 5.5, 600 mM NaCl. Elution fractions are analyzed for their absorbance at 280 nm and the concentration of IFN-β in the samples was estimated using an extinction coefficient of 1.51 for a 1 mg/mL solution. The peak fractions are pooled to give an IFN-β concentration of 2.3 mg/mL. To 1.2 mL of the IFN-β-1a from the SP-Sepharose eluate pool, 0.5 M sodium phosphate pH 6.0 is added to 50 mM, sodium cyanoborohdride (Aldrich) is added to 5 mM, and 20 kDa mPEG-O*-p-*phenylacetaldehyde, is added to 10 mg/mL. The sample is incubated at room temperature for 18 h in the dark. The PEGylated IFN-β can be purified from the reaction mixture on a 0.75 mL SP-Sepharose FF column as follows: 1.5 mL of reaction mixture is diluted with 7.5 mL 20 mM MES pH 5.0, 7.5 mL water, and 5 µL 5 N HCl, and loaded onto the SP-Sepharose column. The column is washed with sodium phosphate pH 5.5, 75 mM NaCl and then the PEGylated IFN-β is eluted from the column with 20 mM MES pH 6.0, 600 mM NaCl. The PEGylated IFN-β is further purified on a Superose 6 HR 10/30 FPLC sizing column with 5 mM sodium phosphate pH 5.5, 150 mM NaCl as the mobile phase. The sizing column (25 mL) is run at 20 mL/h and 0.5 mL fractions are collected. The elution fractions are analyzed for protein content by absorbance at 280 nm, pooled, and the protein concentration of the pool determined. The PEGylated IFN-β concentration is reported in IFN equivalents after adjusting for the contribution of the PEG (20 kDa mPEG-O*-p-*phenylacetaldehyde has an extinction coefficient at 280 nm of 0.5 for a 1 mg/mL solution) to the absorbance at 280 nm using an extinction coefficient of 2 for a 1 mg/mL solution of the PEGylated IFN-β. Samples of the pool can be removed for analysis, and the remainder can be diluted to 30 µg/mL with HSA-containing formulation buffer, aliquoted at 0.25 mL/vial, and stored at -70 °C.

Glycosylated IFN-β coupled to a non-naturally occurring polymer can be used in the methods described. The polymer may comprise a polyalkylene glycol moiety. The polyalkylene moiety may be coupled to the interferon-beta by way of a group selected from an aldehyde group, a maleimide group, a vinylsulfone group, a haloacetate group, plurality of histidine residues, a hydrazine group and an aminothiol group. IFN-β may be coupled to a polyethylene glycol moiety, wherein the IFN-β is coupled to the polyethylene glycol moiety by a labile bond, wherein the labile bond is cleavable by biochemical hydrolysis and/or proteolysis. The polymer may have a molecular weight of from about 5 to about 40 kilodaltons. Another IFN-β that may be used is a physiologically active interferon-beta composition comprising a physiologically active glycosylated interferon-beta N-terminally coupled to a polymer comprising a polyalkylene glycol moiety, wherein the physiologically active interferon-beta and the polyalkylene glycol moiety are arranged such that the physiologically active interferon-beta in the physiologically active interferon-beta composition has substantially similar activity relative to physiologically active interferon-beta lacking said moiety, when measured by an antiviral assay.

Heterologous polypeptides or other molecules can be covalently or non-covalently linked to an IFN-β protein or variant thereof. "Covalently coupled" means that the different moieties are either directly covalently bonded to one another, or else are indirectly covalently joined to one another through an intervening moiety or moieties, such as a bridge, spacer, or linkage moiety or moieties. The intervening moiety or moieties are called a "coupling group." The term "conjugated" is used interchangeably with "covalently coupled."

IFN-βs for use in the invention can be glycosylated or non-glycosylated (or unglycosylated). Non-glycosylated IFN-βs can be produced, e.g., in a prokaryotic host cell.

IFN-β proteins or variants thereof can also be modified by attaching polysaccharides that are not normally present on IFN-βs.

### 3. Methods of producing IFN-β

IFN-β can be produced by any suitable methods, such as methods including constructing a nucleic acid encoding an IFN-β and expressing this nucleic acid in a suitable transformed host. This method will produce recombinant IFN-β. IFN-β may also be produced by chemical synthesis or a combination of chemical synthesis and recombinant DNA technology.

A nucleic acid encoding IFN-β may be constructed by isolating or synthesizing a DNA sequence encoding an IFN-β or variant thereof. For example, an IFN-β fusion protein can be produced as described, e.g., herein. A naturally-occurring IFN-β nucleic acid can be obtained according to methods well known in the art. For example, a nucleic acid can be isolated by reverse transcriptase-polymerase chain reaction (RT-PCR) using RNA obtained from a cell known to express IFN-β, e.g., a leukocyte, and primers based on the sequence of the IFN-β gene, e.g., SEQ ID NO: 1. Nucleic acids encoding IFN-β proteins can also be isolated by screening libraries, e.g., cDNA libraries made from cells expressing IFN-β, with a probe, e.g., an oligonucleotide comprising a portion of an IFN-β sequence.

Alternatively, the complete amino acid sequence may be used to construct a back-translated gene. A DNA oligomer containing a nucleotide sequence coding for IFN-β may be synthesized. For example, several small oligonucleotides coding for portions of the desired polypeptide may be synthesized and then ligated together. The individual oligonucleotides typically contain 5' or 3' overhangs for complementary assembly.

Changes can be introduced into nucleic acids encoding IFN-β proteins by methods well known in the art. For example, changes can be made by site-specific mutagenesis, as described in, e.g., Mark et al., "Site-specific Mutagenesis Of The Human Fibroblast Interferon Gene", Proc. Natl. Acad. Sci. USA, 81, pp. 5662-66 (1984) and U.S. Pat. No. 4,588,585.

Another method of constructing a nucleic acid encoding an IFN-β is via chemical synthesis. For example, a gene that encodes the desired IFN-β may be synthesized by chemical means using an oligonucleotide synthesizer. Such oligonucleotides are designed based on the amino acid sequence of the desired IFN-β .

When choosing a nucleic acid for expression in an expression system, it may be desirable to select those codons that are favored in the host cell or expression system in which the recombinant IFN-β will be produced. It is known, e.g., that certain codons are expressed preferably over others in prokaryotic cells ("codon preference").

A DNA sequence encoding an IFN-β may or may not also include a DNA sequence that encodes a signal sequence. Such signal sequence, if present, should be one recognized by the cell chosen for expression of the IFN-β. The signal sequence may be prokaryotic, eukaryotic or a combination of the two. Signal sequences are well known in the art, and several different ones are described in the art. The signal sequence may be that of a native (i.e., naturally-occurring) IFN-β. The inclusion of a signal sequence depends on whether it is desired to have the IFN-β secreted from the recombinant cells in which it is produced. If the chosen cells are prokaryotic, it generally is preferred that the DNA sequence not encode a signal sequence. If the chosen cells are eukaryotic, it generally is preferred that a signal sequence be encoded and most preferably that the wild-type IFN-β signal sequence be used.

Once assembled (by synthesis, site directed mutagenesis or another method), the nucleic acid encoding an IFN-β is inserted into an expression vector, in which it is operatively linked to an expression control sequence appropriate for expression of the IFN-β in the desired transformed host Proper assembly may be confirmed by nucleotide sequencing, restriction mapping, and expression of a biologically active polypeptide in a suitable host or host cell. As is well known in the art, in order to obtain high expression levels of a transfected gene in a host or host cell, the gene must be operatively linked to transcriptional and translational expression control sequences that are functional in the chosen expression host.

The choice of expression control sequence and expression vector will depend upon the choice of host cell. A wide variety of expression host/vector combinations may be employed. Useful expression vectors for eukaryotic hosts, e.g., eukaryotic host cells, include, for example, vectors comprising expression control sequences from SV40, bovine papilloma virus, adenovirus and cytomegalovirus, e.g., the following vectors: pcDNAI/amp, pcDNAI/neo, pRc/CMV, pSV2gpt, pSV2neo, pSV2-dhfr, pTk2, pRSVneo, pMSG, pSVT7, pko-neo and pHyg derived vectors. Alternatively, derivatives of viruses such as the bovine papillomavirus (BPV-1), or Epstein-Barr virus (pHEBo, pREP-derived and p205) can be used for transient expression of proteins in eukaryotic cells. The various methods employed in the preparation of the plasmids and transformation of host organisms are well known in the art. For other suitable expression systems, see Molecular Cloning A Laboratory Manual, 2nd Ed., ed. by Sambrook, Fritsch and Maniatis (Cold Spring Harbor Laboratory Press: 1989) Chapters 16 and 17.

Useful expression vectors for bacterial hosts include known bacterial plasmids, such as plasmids from E. coli, including col E1, pCR1, pBR322, pMB9 and their derivatives, wider host range plasmids, such as RP4, phage DNAs, e.g., the numerous derivatives of phage lambda, e.g., NM989, and other DNA phages, such as M13 and filamentous single stranded DNA phages. Useful expression vectors for yeast cells include the 2.mu. plasmid and derivatives thereof. Useful vectors for insect cells include pVL 941. See also, Cate et al., "Isolation Of The Bovine And Human Genes For Mullerian Inhibiting Substance And Expression Of The Human Gene In Animal Cells", Cell, 45, pp. 685-98 (1986).

In addition, any of a wide variety of expression control sequences may be used in these vectors. Such useful expression control sequences include the expression control sequences associated with structural genes of the foregoing expression vectors. Examples of useful expression control sequences include, for example, the early and late promoters of SV40 or adenovirus, the lac system, the trp system, the TAC or TRC system, the major operator and promoter regions of phage lambda, for example PL, the control regions of fd coat protein, the promoter for 3-phosphoglycerate kinase or other glycolytic enzymes, the promoters of acid phosphatase, e.g., Pho5, the promoters of the yeast alpha-mating system and other sequences known to control the expression of genes of prokaryotic or eukaryotic cells or their viruses, and various combinations thereof.

Any suitable host may be used to produce IFN-β, including bacteria, fungi (including yeasts), plant, insect, mammal, or other appropriate animal cells or cell lines, as well as transgenic animals or plants. Exemplary hosts include strains of E. coli, Pseudomonas, Bacillus, Streptomyces, fungi, yeast, insect cells such as Spodoptera fruaiperda (SF9), animal cells such as Chinese hamster ovary (CHO) and mouse cells such as NS/0, African green monkey cells such as COS 1, COS 7, BSC 1, BSC 40, and BMT 10, and human cells, as well as plant cells in tissue culture. Such cells can be obtained from the American Type Culture Collection (ATCC). Preferred host cells for animal cell expression include cultured CHO cells and COS 7 cells and particularly the CHO-DDUKY-β1 cell line.

It should of course be understood that not all vectors and expression control sequences will function equally well to express the DNA sequences described herein. Neither will all hosts function equally well with the same expression system. However, one of skill in the art may make a selection among these vectors, expression control sequences and hosts without undue experimentation. The vector's copy number, the ability to control that copy number, and the expression of any other proteins encoded by the vector, such as antibiotic markers, should also be considered. For example, preferred vectors for use include those that allow the DNA encoding the IFN-β to be amplified in copy number. Such amplifiable vectors are well known in the art. They include, for example, vectors able to be amplified by DHFR amplification (see, e.g., Kaufman, U.S. Pat. No. 4,470,461, Kaufman and Sharp, "Construction Of A Modular Dihydrafolate Reductase cDNA Gene: Analysis Of Signals Utilized For Efficient Expression", Mol. Cell. Biol., 2, pp. 1304-19 (1982)) or glutamine synthetase ("GS") amplification (see, e.g., U.S. Pat. No. 5,122,464 and European published application 338,841).

In selecting an expression control sequence, a variety of factors should also be considered. These include, for example, the relative strength of the sequence, its controllability, and its compatibility with the actual DNA sequence encoding the IFN-β , particularly as regards potential secondary structures. Hosts should be selected by consideration of their compatibility with the chosen vector, the toxicity of the product coded for by the DNA sequences , their secretion characteristics, their ability to fold the polypeptides correctly, their fermentation or culture requirements, and the ease of purification of the products coded for by the DNA sequences.

Within these parameters, one of skill in the art may select various vector/expression control sequence/host combinations that will express the desired DNA sequences on fermentation or in large scale animal culture, for example, using CHO cells or COS 7 cells. Use of the CHO cell line CHO-KUKX-B1 DHFR sup for expressing IFN-β variants is further described in U.S. Patent No. 6,127,332.

An IFN-β can also be produced in an *in vitro* system, e.g., in a *in vitro* translation system, e.g., cell lysate, e.g., a reticulocyte lysate. The term "*in vitro* translation system", which is used herein interchangeably with the term "cell- free translation system" refers to a translation system which is a cell-free extract containing at least the minimum elements necessary for translation of an RNA molecule into a protein. *In vitro* translation systems typically comprise macromolecules, such as enzymes, translation, initiation and elongation factors, chemical reagents, and ribosomes. For example, an *in vitro* translation system may comprise at least ribosomes, ^{t}RNAs, initiator methionyl-^{t}RNA^{Met}, proteins or complexes involved in translation, e.g., eIF₂, eIF₃, the cap-binding (CB) complex, comprising the cap-binding protein (CBP) and eukaryotic initiation factor 4F (eIF_{4F}). A variety of *in vitro* translation systems are well known in the art and include commercially available kits. Examples of *in vitro* translation systems include eukaryotic lysates, such as rabbit reticulocyte lysates, rabbit oocyte lysates, human cell lysates, insect cell lysates and wheat germ extracts. Lysates are commercially available from manufacturers such as Promega Corp., Madison, Wis.; Stratagene, La Jolla, Calif.; Amersham, Arlington Heights, III.; and GIBCO/BRL, Grand Island, N.Y. RNA for use in *in vitro* translation systems can be produced *in vitro,* e.g., using SP6 or T7 promoters, according to methods known in the art.

In another method, an IFN-β is expressed from the endogenous gene in a host cell. The method may comprise inserting a heterologous promoter upstream of the coding region of the IFN-β gene, e.g., an inducible promoter, expressing the endogenous IFN-β gene and recovering the IFN-β produced. A heterologous promoter can be introduced into cells by "knock-in," according to methods known in the art, or alternatively, by insertion of the promoter within the IFN-β gene.

The IFN-β obtained may be glycosylated or unglycosylated depending on the host organism used to produce it . If bacteria are chosen as the host, then the IFN-β produced will be unglycosylated. Eukaryotic cells, on the other hand, will glycosylate the IFN-β.

The IFN-β produced by the transformed host can be purified according to any suitable method. Various methods are known for purifying IFN-β. See, e.g., U.S. Pat. Nos. 4,289,689, 4,359,389, 4,172,071, 4,551,271, 5,244,655, 4,485,017, 4,257,938, 4,541,952 and 6,127,332. Preferably, the IFN-β is purified by immunoaffinity, as described, e.g., in Okamura et al., "Human Fibroblastoid Interferon: Immunosorbent Column Chromatography And N-Terminal Amino Acid Sequence." Biochem., 19, pp. 3831-35 (1980).

For example, the IFN-β proteins and variants thereof may be isolated and purified in accordance with conventional conditions, such as extraction, precipitation, chromatography, affinity chromatography, electrophoresis or the like. For example, the interferon proteins and fragments may be purified by passing a solution thereof through a column having an interferon receptor immobilized thereon (see U.S. Pat. No. 4,725,669). The bound interferon molecule may then be eluted by treatment with a chaotropic salt or by elution with aqueous acetic acid. The immunoglobulin fusion proteins may be purified by passing a solution containing the fusion protein through a column which contains immobilized protein A or protein G which selectively binds the Fc portion of the fusion protein. See, for example, Reis, K, J., et al, J. Immunol. 132:3098-3102 (1984); PCT Application, Publication No. W087/00329. The chimeric antibody may then be eluted by treatment with a chaotropic salt or by elution with aqueous acetic acid.

Alternatively the interferon proteins and immunoglobulin-fusion molecules may be purified on anti-interferon antibody columns, or on anti-immunoglobulin antibody columns to give a substantially pure protein. By the term "substantially pure" is intended that the protein is free of the impurities that are naturally associated therewith. Substantial purity may be evidenced by a single band by electrophoresis.

IFN-β that has been produced and purified can be characterized, e.g., by peptide mapping. For example, an IFN-β sample can be digested with endoproteinase Lys-C and analyzed on a reverse phase HPLC, as described, e.g., in U.S. Patent No. 6,127,332.

Preferably, the IFN-β is substantially free of other cellular material, e.g., proteins. The terms "substantially pure" or "purified preparations of an IFN-β" refers to preparations of the IFN-β having less than about 20% (by dry weight) contaminating cellular material, e.g., nucleic acids, proteins, and lipids, and preferably having less than about 5% contaminating cellular material. Preferred preparations of the IFN-β have less than about 2% contaminating cellular material; even more preferably less than about 1% contaminating cellular material and most preferably less than about 0.5; 0.2; 0.1; 0.01; 0.001% contaminating cellular material.

Preferred IFN-β compositions are also substantially free of other cellular proteins (also referred to herein as "contaminating proteins"), i.e., the compositions have less than about 20% (by dry weight) contaminating protein, and preferably having less than about 5% contaminating protein. Preferred preparations of the subject polypeptides have less than about 2% contaminating protein; even more preferably less than about 1% contaminating protein and most preferably less than about 0.5; 0.2; 0.1; 0.01; 0.001% contaminating proteins.

The purity and concentration of IFN-β preparations can be determined according to methods known in the art, e.g., by subjecting samples to gel electrophoresis, and as described, e.g., in Robert K. Scopes, Protein Purification, Principles and Practice, Third Ed., Springer Verlag New York, 1993, and references cited therein.

The biological activity of IFN-β can be assayed by any suitable method known in the art, e.g., antibody neutralization of antiviral activity, induction of protein kinase, oligoadenylate 2,5-A synthetase or phosphodiesterase activities, e.g., as described in EP-B1-41313 and WO 00/23472. Such assays also include immunomodulatory assays (see, e.g., U.S. Pat. No. 4,753,795), growth inhibition assays, and measurement of binding to cells that express interferon receptors. Exemplary antiviral assays are further described in U.S. Patent 6,127,332 and WO 00/23472.

The ability of IFN-β to treat glomerulonephritis can also be assessed in animal models, e.g., those described in the Examples and further herein. The testing can be conducted, e.g., as described in the Examples.

IFN-β can also be purchased commercially under the following brand names: AVONEX® (IFN-β-1a) (Biogen, Inc., Cambridge, MA); REBIF^{®} (IFN-β-1a) (Serono, S.A., Geneva, Switzerland); and BETAFERON^{®} (IFN-β-1b) (Schering Aktiengesellschaft, Berlin, Germany), which is also marketed as BETASERON^{®} (Berlex, Montville, NJ; IFN-β-1b). AVONEX^{®} and REBIF^{®} are recombinant wild-type human glycosylated IFN-β produced in Chinese hamster ovary cells. BETAFERON^{®} is produced in bacteria.

### 4. Methods of treatment with IFN-β

The invention provides an IFN-β for use in methods for treating or preventing a chronic demyelinating motor neuropathy in a subject, comprising intramuscularly administering to the subject a therapeutically effective amount of IFN-β , optionally as an adjunct to another therapy. Examples of chronic demyelinating neuropathies include CIDP and multifocal motor neuropathy. In a preferred embodiment, the neuropathy is CIDP.

The subject may be a subject who has been identified as having a neuropathy. A subject can be diagnosed with a neuropathy according to methods known in the art. In particular, diagnosis of CIDP can be done according to methods known in the art, e.g., as further described herein. The treatment with an IFN-β can be started at any time in a person diagnosed with the neuropathy. A treatment can also be started in a subject that does not appear to have the neuropathy, but is likely to develop it Such subjects can be identified, e.g., by genetic criteria. Subjects likely to develop the neuropathy also include subjects having some but not all symptoms typically associated with the neuropathy, such that in certain instances it may not be clear whether the subject will in fact develop the neuropathy. Treatments can be conducted for at least about 1 month, at least about 3 months, at least about 6 months, at least about 1 year, at least about 3 years, at least, about 5 years or longer.

A subject can be an animal, such as a mammal Examples of mammals include humans, bovines, ovines, porcines, equines, canines, felines, non-human primates, mice and rats.

In certain embodiments, an IFN-β- is administered to a subject as an adjunct therapy, i.e., to a subject who is also receiving another treatment. For example, a person having CIDP may be treated by the administration of IVIg; steroids, such as prednisolone; or an immunosuppressive agent, such as azothioprine, cyclosporin or cyclophosphamide; or by plasma exchange, in addition to receiving IFN-β. Combination therapy with IFN-β may minimize the use of the other treatment, which other treatment may be more harmful (e.g., steroids), more expensive (e.g., IVIg) or more inconvenient (plasma exchange). Accordingly, in certain embodiments, administration of an IFN-β to a subject permits one to decrease the dose and/or frequency of the other treatment For example, doses and/or frequencies can be reduced by at least about 10%, 30%, 50%, 75%, 100% (i.e., two fold), five fold, 10 fold or more. Set forth below, are current standards of care for CIDP.

Accordingly, in one instance, the disclosure provides the treatment of a chronic demyelinating neuropathy, e.g., CIDP in a subject receiving a first CIDP treatment selected from the group consisting of administration of a steroid; administration of an anti-inflammatory drug; administration of IVIg; and plasmapheresis, the improvement comprising administering to the subject, in addition to the first CIDP treatment, a dose of an intramuscularly administered IFN-β therapeutic in an amount effective to significantly reduce the dose or frequency of the first CIDP treatment, to provide effective relief from symptoms of CIDP.

Immunosuppressants are currently used for treating CIDP. For example, steroids have been found to be beneficial in CIDP. A favorable response is usually seen within 4 weeks. One steroid that is commonly used is Prednisone (Deltasone, Orasone, Meticorten). Prednisone is an oral corticosteroid that suppresses inflammation and immune responses and is believed to alter mediator function at site of inflammation and suppressing immune responses in CIDP. Although doses vary; most adult patients are started on 0.5 to 1 mg/kg/day PO (i.e., by mouth) initially (about 30-40 to 60-80 mg/day). Improvement can be anticipated within the next 2 months. Later, the dosing may be converted to alternate-day treatment and then titrated to lowest effective dose that allows maintaining a patient in remission.

Another immunosuppressant that have been found effective in treating CIDP is Azathioprine (Imuran), which is a purine analog that decreases metabolism of purines and also may inhibit DNA and RNA synthesis. Azathioprine is believed to reduce disability and symptoms of CIDP by suppressing immune-mediated damage to nerves. The initial dose is about 50 mg PO qd (by mouth, daily), which is usually increased gradually to a total daily dosage of 2-3 mg/kg/day PO. Although therapeutic doses of azathioprine are difficult to determine for each patient; some evidence suggests that elevations of red blood cell volume (MCV) indicate therapeutic dosing. Therapeutic responses may take more than 6 months to become apparent.

Yet another immunosuppressant that is currently used for treating CIDP is Mycophenolate (CellCept), which is a prodrug for immunosuppressive agent mycophenolic acid. Mycophenolate is believed to Inhibit lymphocyte purine synthesis by inhibiting enzyme inosine monophosphate dehydrogenase. The typical dose for an adult is 250 mg to 3 g/day, with an adjustment of the dose depending on clinical effect.

Cyclosporine (Sandimmune, Neoral), which is a cyclic polypeptide consisting of 11 amino acids can also be used for treating CIDP. Cyclosporine inhibits first phase of T cell activation and does not affect humoral immunity. It is believed that by suppressing T cells, cyclosporine may inhibit cell-mediated nerve damage at site of inflammatory/immune reaction. Usually, it is administered at 5 mg/kg/day PO divided bid (by mouth twice a day) initially and the dose is increased according to the response. Trough and peak levels should be monitored to register efficacy and avoid toxicity; although no definitive desirable trough level has been identified specifically for CIDP, usual trough levels utilized for immunologic disorders are between 100 and 250.

Another immunosuppressant is cyclophosphamide (Cytoxan), which is a cell-cycle phase-nonspecific antineoplastic agent and immunosuppressant that acts as alkylating agent. The dose is typically 1-2 mg/kg/d PO.

Another standard treatment of CIDP patients is by intravenous immunoglobulin (IVIg) administration. The solution for IV infusion is typically composed mostly of heterogenous human IgG but also small amounts of IgA and IgM. In certain cases, the IV solution is heterogeneous with regard to the epitopes recognized by the antibodies, e.g., it is not a preparation of antibody obtained from immunization of an animal with a particular antigen. Its proposed mechanism of action is based on the thought that IVIg contains random sets of antibodies that would neutralize immune factors, causing damage to peripheral nerve in CIDP. On average, improvement is seen by day 10 and continues through day 42. The serum half-life approximately 21-29 days. Patients usually require repeated treatments every few weeks or months to maintain remission or treat recurrences. The common does is between 0.4 and 2 g/kg, usually divided into as many as 5 daily doses of 400 mg/kg. Treatment may be initiated at a higher dose, e.g., 2g/kg and later reduced to a lower dose, e.g., 0.5 to 1 g/kg The frequency of administration of these doses varies, with most patients receiving doses every 2 to 8 weeks. For example, IVIG can be administered at 0.4 g/kg over several days to 1 to 2 g/kg every 1 to 4 weeks.

Another approved treatment of CIDP is plasmapheresis (or plasma exchange). This treatment is believed to remove antibodies and complement components that are responsible for immune-mediated damage of peripheral nerves. The plasma is removed from the blood through a method similar to dialysis. Its efficacy appears to be similar to that of IVIg in treatment of CIDP. Commonly, patients undergo 3 plasma exchanges per week for the first two weeks; after that, the number and frequency of treatments is determined by clinical response.

Accordingly, an IFN-β can be administered to a subject together with administration of an immunosuppressant, e.g., a steroid; with administration of IVIg and/or with plasmapheresis. The IFN-β and the adjunct drug can be administered simultaneously or consecutively. If consecutively, it may be administered the same day or on different days. When an IFN-β therapy is combined with plasmapheresis, the IFN-β may be administered after the plasmapheresis, such that no IFN-β is removed during the plasmapheresis. When an IFN-β therapy is combined with an IVIg therapy, administration of the two different drugs is preferably separated by at least one or two hours.

In another embodiment, an IFN-β is administered to subjects who were found to be refractory to another CIDP therapy, such as those set forth above. In other situations, an IFN-β is administered to subjects who were not found to be refractory to another CIDP therapy. For example, an IFN-β may be administered to subjects who were responsive to one or more other therapies. In yet other embodiments, an IFN-β is administered to subjects who are naïve, i.e., have not had any treatment for CIDP previously. When a subject had not previously received treatment for CIDP, the methods of treatment with an IFN-β may comprise first identifying a subject as having CIDP or as likely to develop CIDP.

A treatment may also be as follows: an IFN-β is administered to a parson receiving a first CIDP treatment, such as IVIG, and the combination treatment is continued for a certain amount of time, following which the first CIDP treatment is discontinued. The first CIDP treatment may be discontinued progressively, such as by reducing the dose and/or frequency of the first CIDP treatment. In an illustrative embodiment, a subject is receiving IVIG once every two weeks or once every four weeks. The subject then receives, in combination with the IVIG treatment, an IFN treatment, such as weekly or biweekly administration of an IFN-β. The combined treatment may be continued for about 10-20 weeks, e.g., about 16 weeks. During the combined treatment, it may be preferable to separate the administrations of IVIg and the IFN-β by at least about 1 to 3 hours, such by at least about 2 hours. After about 16 weeks, the first CIDP treatment is interrupted or reduced. For example, the subject may then receive smaller doses of IVIg or less frequent administrations. In individuals who do not improve following the discontinuation or reduction of the first CIDP treatment, the first CIDP treatment may be reinstated and a combination treatment with an IFN-β pursued.

Generally, IFN-β may be administered by any route. For example, IFN-β may be provided to an individual directly, e.g., locally, as by injection or topical administration to a tissue locus or systemically, e.g., parenterally or orally. Local administration includes, e.g., administration directly into an affected muscle. Parenteral administration includes aerosol, subcutaneous, intravenous, intramuscular, intra-articular, intrasynovial, intraperitoneal, intrasternal, intrathecal, intrahepatic, intralesional and intracranial injection or infusion techniques. Administration may be by periodic injections of a bolus of IFN-β, or it may be made more continuous by intravenous or intraperitoneal administration from a reservoir which is external (e.g., an i.v. bag) or internal (e.g., a bioerodable implant or implanted pump).

The IFN-β are preferably administered as a sterile pharmaceutical composition containing a pharmaceutically acceptable carrier. The term "carrier" as used herein includes acceptable adjuvants and vehicles. Pharmaceutically acceptable carriers that may be used in the pharmaceutical compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as prolamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol, wool fat, dextrose, glycerol, ethanol and the like or combinations thereof. IFN-β may be prepared in a composition comprising one or more other proteins, e.g., for stabilizing the IFN-β. For example, IFN-β can be mixed with albumin,

Where the IFN-β is to be provided parenterally the agent preferably comprises part of an aqueous solution. The solution is physiologically acceptable so that in addition to delivery of the desired IFN-β to the subject, the solution does not otherwise adversely affect the subject's electrolyte and/or volume balance. The aqueous medium for the IFN-β thus may comprise normal physiologic saline (e.g., 0.9% NaCl, 0.15M, pH 7-7.4). Useful solutions for parenteral administration may be prepared by any of the methods well known in the pharmaceutical art, described, for example, in Remington's Pharmaceutical Sciences (Gennaro, A., ed), Mack Pub., 1990.

Pharmaceutical compositions may be in the form of a sterile injectable preparation, for example a sterile injectable aqueous or oleaginous suspension. This suspension may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as do natural pharmaceutically-acceptable oils, such as olive oil or castor oil, especially in their polyoxyethylated versions. These oil solutions or suspensions may also contain a long-chain alcohol diluent or dispersant.

Pharmaceutical compositions comprising IFN-β may also be given orally. For example, they can be administered in any orally acceptable dosage form including, but not limited to, capsules, tablets, aqueous suspensions or solutions. In the case of tablets for oral use, carriers that are commonly used include lactose and corn starch. Lubricating agents, such as magnesium stearate, are also typically added. For oral administration in a capsule form, useful diluents include lactose and dried corn starch. When aqueous suspensions are required for oral use, the active ingredient is combined with emulsifying and suspending agents. If desired, certain sweetening, flavoring or coloring agents may also be added. Topically-transdermal patches may also be used.

The pharmaceutical compositions may also be administered by nasal aerosol or inhalation through the use of a nebulizer, a dry powder inhaler or a metered dose inhaler. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

IFN-β can be administered in the form of liposome delivery systems, such as small unilamellar vesicles, large unilamellar vesicles and multilamellar vesicles. Liposomes can be formed from a variety ofphospholipids, containing cholesterol, stearylamine, or phosphatidylcholines. A film of lipid components may be hydrated with an aqueous solution of drug to a form lipid layer encapsulating the drug, as described in U.S. Pat No. 5,262,564. Liposomes may contain surface molecules that direct them to particular cells or tissues. Such modified liposomes can be prepared according to methods known in the art.

IFN-βs or variants thereof may also be coupled to soluble polymers as targetable drug carriers. Such polymers can include polyvinylpyrrolidone, pyran copolymer, - polyhydroxypropyl-methacrylamide-phenol, polyhydroxyethylaspanamidephenol, or polyethyleneoxidepolylysine substituted with palmitoyl residues. IFN-βs or variants thereof can also be coupled to proteins, such as, for example, receptor proteins and albumin. Furthermore, the IFN-βs or variants thereof may be coupled to a class of biodegradable polymers useful in achieving controlled release of a drug, for example, polylactic acid, polyepsilon caprolactone, polyhydroxy butyric acid, polyorthoesters, polyacetals, polydihydropyrans, polycyanoacrylates and cross-linked or amphipathic block copolymers of hydrogels.

An IFN-β may also be provided as a liquid composition comprising a stabilizing agent The stabilizing agent may be present at an amount of between 0.3% and 5% by weight of the IFN-β. The stabilizing agent may be an amino acid, such as an acidic amino acid (e.g., glutamic acid and aspartic acid) or arginine or glycine. If the stabilizing agent is arginine-HCl, its concentration will preferably range between 0.5% (w/v) to 5% and is most preferably 3.13% (equivalent to 150 mM arginine-HCl). If the stabilizing agent is glycine, its concentration will preferably range between 0.5% (w/v) to 2.0% and most preferably 0.52% (equivalent to 66.7 mM to 266.4 mM, and most preferably 70 mM). If the stabilizing agent is glutamic acid, its concentration will preferably range between 100 mM to 200 mM, and is most preferably 170 mM (equivalent to a w/v percent ranging from 1.47% to 2.94% and most preferably 2.5%). In certain cases, the range of concentrations of IFN-β in the liquid formulations is from about 30 µg/ml to about 250 µg/ml, such as 48 to 78 µg/ml, e.g., about 60 µg/ml. This amount will depend, e.g., on the specific activity of the particular IFN-β. Generally, the range of doses will be from about 1 million international unit (MIU) to about 50 MIU, e.g., about 3, 6, 9, or 12 MIU per dose.

The amino acid stabilizing agent is may be arginine, which is incorporated as its acidic form (arginine-HCl) in about pH 5.0 solutions. Accordingly, poly-ionic excipients are preferred in this instance. The liquid composition may be contained within a vessel, e.g., a syringe, in which the vessel has a surface in contact with the liquid that is coated with a material that is inert to IFN-β, e.g., silicone or polyetrafluoroethylene. Preferred compositions have a pH between 4.0 and 7.2. In certain cases, the solution comprising the stabilizing agent has not been lyophilized and/or has not been subject to oxygen containing gas during preparation and storage.

Organic acid and phosphate buffers to be used to maintain the pH in the range of about 4.0 to about 7.2, such as from about 4.5 to about 5.5, e.g., 5,0, can be conventional buffers of organic acids and salts thereof such as citrate buffers (e.g., monosodium citrate-disodium citrate mixture, citric acid-trisodium citrate mixture, citric acid-monosodium citrate mixtures, etc.), succinate buffers (e.g., succinic acid-monosodium succinate mixture, succinic acid-sodium hydroxide mixture, succinic acid-disodium succinate mixture, etc.), tartrate buffers, fumarate buffers, gluconate buffers, oxalate buffers, lactate buffers, phosphate buffers, and acetate buffers, as further described in WO 98/28007.

Exemplary formulations, which can be prepared as described in WO 98/38007, include:
(i) a 20 mM acetate buffer at pH 5.0, the buffer having preferably not previously been lyophilized, in which the buffer includes IFN-β and at least one ingredient selected from (a) 150 mM arginine-HCl; (b) 100 mM sodium chloride and 70 mM glycine; (c) 150 mM arginine-HCl and 15 mg/ml human serum albumin; (d) 150 mM arginine-HCl and 0.1% Pluronic F-68; (e) 140 mM sodium chloride; (f) 140 mM sodium chloride and 15 mg/ml human serum albumin; and (g) 140 mM sodium chloride and 0.1 % Pluronic F-68;
(ii) a liquid at pH 5.0 that includes IFN-β or a variant thereof, 170 mM L-glutamic acid, and 150 mM sodium hydroxide, the liquid preferably not having previously been lyophilized; and
(iii) a 20 mM phosphate buffer at pH 7.2, the buffer having preferably not previously been lyophylized, wherein the buffer includes IFN-β and least one ingredient selected from: (a) 140 mM arginine-HCl and (b) 100 mM sodium chloride and 70 mM glycine.

Prefered compositions also include polysorbate, e.g., at 0.005% w/v polysorbate 20.

IFN-β or a variant thereof can also be administered together with a soluble IFN type I receptor or portion thereof, such as an IFN-binding chain of the receptor, as described, e.g., in U.S. Patent No. 6,372,207. As described in the patent, administration of an IFN type I in the form of a complex with an IFN binding chain of the receptor improves the stability of the IFN and enhances the potency of the IFN. The complex may be a noncovalent complex or a covalent complex.

IFN-βs can be formulated in dry powder form, which may or may not be solubilized or suspended prior to administration to a subject. In particular, it has been shown that IFN-βs conjugated to a polymer, e.g., PEG are particularly stable in dry form (see, e.g., WO 00/23114 and PCT/US/95/06008).

The formulated compositions may contain therapeutically effective amounts of an IFN-β, i.e., they may contain amounts of an IFN-β that provides appropriate concentrations of the IFN-β to the muscular tissues or other appropriate tissues for a time sufficient to prevent, inhibit, delay or alleviate permanent or progressive loss of muscular function, or otherwise provide therapeutic efficacy. As will be appreciated by those skilled in the art, the concentration of the IFN-β in a therapeutic composition may vary depending upon a number of factors, including the biological efficacy of the selected IFN-β, the chemical characteristics (e.g., hydrophobicity) of the IFN-β employed, the formulation of the IFN-β excipients, the administration route, and the treatment envisioned, such as whether the IFN-β will be administered directly into a tissue or whether it will be administered systemically. The preferred dosage to be administered may also depend on such variables as the conditions of the tissues of the subject, the extent of muscular loss, and the overall health status of the particular subject. It may also depend on the age, weight, sex, general health, diet rate of excretion of the patient, as well as the sensitivity of the subject to side effects and whether the IFN-β is coadministered with other drugs. An ordinarily skilled physician or veterinarean can readily determine and prescribe the effective amount of the IFN-β required to prevent, counter or arrest the progres of the condition.

Dosages may be administered continuously, or daily, but it is currently preferred that dosages be administered once, twice or three times per week for as long as a satisfactory response persists (as measured, for example, by stabilization and/or improvement of the disease by appropriate medical markers and/or quality of life indices). Less frequent dosages, for example monthly dosages, may also be employed. In order to facilitate frequent infusions, implantation of a semi-permanent stent (e.g., intravenous, intraperitoneal or intracapsular) may be advisable.

Any of the above pharmaceutical compositions may contain 0.1-99%, 1-70%, or, preferably, 1-50% of IFN-β as active ingredients.

For any route of administration, divided or single doses may be used. For example, IFN-β may be administered daily or weekly, in a single dose, or the total dosage may be administered in divided doses of two, three or four.

IFN-β may be administered at dosage levels of between about 0.001 and about 100 mg/kg body weight per dose, e.g., between about 0.1 and about 50 mg/kg body weight; between about 0.1 mg/kg body weight and about 20 mg/kg body weight; or between about 1 mg/kg body weight and about 3 mg/kg body weight. These doses may be administered at intervals of every 1-14 days, such as every day, every other day, every third day, every fifth day, weekly, or biweekly. Depending in particular on the specific activity of the IFN-β, it may also be administered at a dose ranging from about 10 to about 100 µg/dose, such as from about 20 to about 50µg/dose, e.g., at about 30µg/dose.

In terms of international units, an IFN-β may be administered at a dose of between about 1 and 30 MIU/dose, such as between 3 and 20 MIU/dose, e.g., between 3 and 12 MIU. Preferred doses include about 3 MIU, 6 MIU and 12 MIU per dose. Such doses are preferably administered about once or twice weekly. Optimization of dosages can be determined, e.g., by administration of the IFN-β, followed by assessment of the circulating or local concentration of the IFN-β.

An IFN-β may be administered by subcutaneous injection to deliver 0.01-100 µg/kg, or more preferably 0.01-10 µg/kg of IFN-β, e.g., PEGylated IFN-β, over one week, two injections of 0.005-50 µg/kg, or more preferably 0.005-5 µg/kg, respectively, may be administered at 0 and 72 hours. Additionally, one approach for parenteral administration employs the implantation of a slow-release or sustained-released system, which assures that a constant level of dosage is maintained, according to U.S. Pat. No. 3,710,795.

Oral dosages, preferably for pegylated IFN-β, will range between about 0.01-100 µg/kg/day orally, or more preferably 0.01-10 µg/kg/day orally. The compositions are preferably provided in the form of scored tablets containing 0.5-5000 µg, or more preferably 0.5-500 µg of IFN-β. In a preferred embodiment, a subject having a neuropathy, e.g., CIDP is treated with an IFN-β by intramuscular administration of the IFN-β. The IFN-β may be administered weekly. In an even more preferred embodiment, the IFN-β is administered weekly to the subject at a dose of about 6 MIU. In other embodiments, a subject is treated by weekly administration of about 6 MIU of IFN-β, which administration is not necessarily intramuscular.

In certain cases an IFN-β is administered according to one regimen for a certain interval of time, and is then administered according to another regimen. For example, a subject may receive an IFN-β weekly for two months and then twice weekly for the following months. A subject can also be treated by subcutaneous administration first and then by intramuscular administration. In other regimens, a particular dose, mode of administration or IFN is alternated with a different dose, mode of administration or IFN for every other administration.

Preferably the IFN-β is AVONEX^{®}. AVONE7^{®} is sold as a lyophilized powder consisting of the following:
Formulation per 1ml dose:
   30 mcg interferon-b-1a (6 million international units (MIU))
   50mM sodium phosphate
   100mM sodium chloride
   15mg Human Serum Albumin
   pH 7.2
The specific activity of AVONEX^{®} interferon is 2 x 10⁸ units/mg, i.e., 200 MU of antiviral activity per milligram of IFN-b-1a protein. The patient reconstitues the powder with sterile water prior to intramuscular injection of the 1 ml once per week. AVONEX^{®} can also be prepared as a liquid formulation consisting of the following:
Formulation per 0.5ml dose:
   30 mcg (µg) IFN-b-1a (6 million international units (MIU))
   20 mM acetate (sodium acetate and acetic acid)
   150 mM arginine HCl
   0.005% w.v polysorbate 20
   water for injection
   pH 4.8
This formulation can be packaged in a pre-filled syringe. The patient may either manually use the syringe as provided or use in conjunction with an autoinjector. The dosing schedule is 6 MIU (i.e., 30 mcg) intramuscular once per week.

The IFN-β may also be REBIF®, which is provided as a lyophilized powder and as a liquid formulation. The lyophilized powder consists of the following:
Formulation per 2.0ml dose:
   3 MIU of IFN-b-1a
   mannitol
   HSA
   Sodium acetate
   pH 5.5
The specific activity of REBIF® interferon is 2.7 x 10⁸ units/mg, i.e., 270 MU of antiviral activity per milligram of IFN-b-1a protein. The patient reconstitutes the powder with a sodium chloride solution (0.9% NaCl) prior to injection subcutaneously three times a week. The formulation of liquid REBIF® is as follows:
Formulation per 0.5 ml dose:
   6 or 12 MIU IFN-b-1a
   4 or 2 mg HSA
   27.3 mg mannitol
   0.4 mg sodium acetate
   water for injection
The liquid formulation is packaged in a pre-filled syringe and administered with or without use of an autoinjector device (Rebiject) 3 times (6 or 12 MIU, corresponding to 66 µg/week or 132 µg/week, respectively) per week subcutaneously.

Furthermore, the IFN-β is may be BETASERON^{®} (from Berlex), an IFN-β containing a cys-17 to ser mutation that is produced in *E*. *coli.* This non-glycosylated IFNβ is less potent than AVONEX^{®} or REBIF^{®} which are both produced in CHO cells. Doses are sold as 250 mcg (8 MIU) doses, both in lyophilized and liquid formulations, for injection subcutaneously every other day. BETAFERON^{®} is another commercially available IFN-β, which can be administered subcutaneously, according to the manufacturer's instructions.

In addition to an IFN-beta, and optionally another therapy, subjects may also receive medication for the treatment of neuropathic pain, e.g., antiepileptic medications. The two most frequently used medications are gabapentin (Neurontin) and carbamazepine (Tegretol). Alternatively, tricyclic antidepressants, e.g., amitriptyline (Elavil), can also be used for the treatment of neuropathic pain.

The course of the disease and its response to drug treatments may be followed by clinical examination and laboratory findings. The effectiveness of the therapy is determined by the extent to which the previously described signs and symptoms of the disease are alleviated and the extent to which the normal side effects of interferon (*i.e.*, flu-like symptoms such as fever, headache, chills, myalgia, fatigue, etc. and central nervous system related symptoms such as depression, paresthesia, impaired concentration, etc.) are eliminated or substantially reduced.

The present invention is further illustrated by the following examples, which should not be construed as limiting in any way.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning A Laboratory Manual, 2nd Ed., ed. by Sambrook, Fritsch and Maniatis (Cold Spring Harbor Laboratory Press: 1989); DNA Cloning, Volumes I and II (D. N. Glover ed., 1985); Oligonucleotide Synthesis (M. J. Gait ed., 1984); Mullis et al. U.S. Patent No: 4,683,195; Nucleic Acid Hybridization(B. D. Hames & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols.154 and 155 (Wu et al. eds.), Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); Manipulating the Mouse Embryo, (Cold Spring Harbor Laboratory Press, Cold Spring Harbor, N.Y., 1986).

### Examples

### Example 1: Treatment of CIDP patients with IFN-β1a

CIDP patients that are being treated with IVIg will be switched to an IFN-β1a treatment as follows

Patients having an established diagnosis for CIDP and who are being treated with a stable once every two weeks or once every four weeks regimen of IVIgIVIGIVIg will be given one of the following regimens of IFN-β 1a via intramuscular injection: 30 mcg (6 MIU) of AVONEX^{®} once weekly; 30 mcg (6 MIU) of AVONEX^{®} twice weekly; 60 mcg (12 MIU) of AVONEX^{®} once weekly; or 60 mcg (12 MIU) of AVONEX^{®} twice weekly. When administration of IVIg and IFN-β 1a fall on the same day, the administrations will be separated by at least a two hour period. The patients will receive this combination treatment for 16 weeks, during which time, the disease state of the patients will be assessed about every four weeks. At week 16, the IVIg will be discontinued and the IFN-β 1a treatment will be continued following the same regimen as before.

The disease will continue to be monitored in the patients. If patients were doing better with the combination treatment, the IVIg treatment will be reinstated. IVIg may later be reduced by progressively decreasing the amount or frequency of IVIg administrations.

## Claims

1. Use of an IFN-β in the manufacture of a medicament for the treatment of a chronic demyelinating motor neuropathy in a mammal, wherein the IFN-β is to be administered intramuscularly.

2. The use of claim 1, wherein the chronic demyelinating motor neuropathy is chronic inflammatory demyelinating neuropathy (CIDP).

3. The use of claim 1 or claim 2, wherein the IFN-β comprises mature IFN-β.

4. The use of any one of claims 1-3, wherein the IFN-β is mature IFN-β That lacks the first methione.

5. The use of any one of claims 1-4, wherein the IFN-β is human IFN-β.

6. The use of claim 1, wherein the IFN-β is at least 95% identical to full length mature human IFN-β having SEQ ID NO: 4.

7. The use of claim 6, wherein the IFN-β comprises SEQ ID NO: 4.

8. The use of any one of claims 1-7, wherein the IFN-β is glycosylated.

9. The use of any one of claims 1-7, wherein the IFN-β is not glycosylated

10. The use of claim 1, wherein the IFN-β is IRN-β-1a.

11. The use of claim 1, wherein the IFN-β is IFN-β-1b.

12. The use of any one of claims 1-11, wherein the IFN-β is fused to the constant domain of an immunoglobulin molecule.

13. The use of claim 12, wherein the immunoglobulin molecule is a human immunoglobulin molecule.

14. The use of claim 13, wherein the immunoglobulin molecule is the heavy chain of IgG1.

15. The use of claim 14, wherein the fusion of the IFN-β to the constant domain of an immunoglobulin molecule comprises SEQ ID NO: 14.

16. The use of any one of claims 1-15, wherein the IFN-β comprises a pegylated IFN-β.

17. The use of any one of claims 1-16, wherein the medicament comprises a stabilizing agent.

18. The use of claim 17, wherein the stabilizing agent is an acidic amino acid.

19. The use of claim 18, wherein the stabilizing agent is arginine.

20. The use of any one of claims 1-19, wherein the medicament has a pH between 4.0 and 7.2.

21. The use of any one of claims 1-20, wherein the treatment comprises to the mammal an IFN-β at intervals of every 1-14 days.

22. The use of claim 21, wherein the IFN-β is to be administered weekly at a dose of 6 MIU.

23. The use of claim 21, wherein the IFN-β is to be administered twice a week at a dose of 6 MIU.

24. The use of claim 21, wherein the IFN-β is to be administered weekly at a dose of 12 MIU.

25. The use of claim 21, wherein the IFN-β is to be administered twice a week at a dose of 12 MIU.

26. The use of any one of claims 1-25, wherein the mammal is a human.

27. The use of any one of claims 1-26 wherein the medicament is to be administered to a subject who has not previously been found to be resistant to other treatments for the chronic demyelinating neuropathy.

28. The use of any one of claims 1-27, wherein the medicament is to be administered in a combination treatment comprising an immunosuppressant or plasmapheresis.

29. The use of claim 28, wherein the medicament is to be administered in a combination treatment comprising an immunosuppressant selected from the group consisting of a steroid, azothioprine, cyclosporin, cyclophosphamide, and mycophenolate.

30. the use of any one of claims 1-27, wherein the chronic demyelinating motor neuropathy is chronic inflammatory demyelinating neuropathy (CIDP), and wherein the medicament is to be administered in a combination treatment comprising a second CIDP treatment selected from the group consisting of administration of Mg; administration of a steroid; administration of an anti-inflammatory drug and Plasmapheresis.

31. The use of claim 30, wherein the IFN-β is to be administered weekly.

32. The use of any one of claims 1-27, wherein the medicament is to be administered to a subject being treated with another treatment for a chronic demyelinating motor neuropathy selected from the group consisting of administration of Mg; administration of a steroid; administration of an anti-inflammatory drug and Plasmapheresis and the treatment further comprises phasing out the other treatment.

33. An IFN-β, for USB in treating a chronic demyelinating motor neuropathy in a mammal, wherein the IFN-β is to be administered intramuscularly.

34. An IFN-β, for use in a method of treating CIDP, wherein the method comprises administering to a subject having CIDP a pharmaceutically effective amount of an IFN-β via an intramuscular route, and further administering to the subject an immunosuppressant or subjecting the subject to plasmapheresis.

35. The IFN-β for use in a method of treating CIDP of claim 34, wherein the method of treating CIDP comprises immunosuppressant selected from the group consisting of a steroid, azothioprine, cyclosporin, cyclophosphamide, and mycophenolate.

36. An IFN-β, for use in a method of treating CIDP, wherein the method comprises administering to a subject having CIDP a pharmaceutically effective amount of an IFN-β in combination with a second CIDP treatment selected from the group consisting of administration IVIg; administration of a steroid; administration of an anti-inflammatory drug and plasmapheresis, and wherein administration of the IFN-β is via an intramuscular route.

37. The IFN-β for use in a method of treating CIDP of claim 36, wherein the administration of the IFN-β is weekly.

## Patentansprüche

1. Verwendung eines IFN-β zur Herstellung eines Arzneimittels für die Behandlung einer chronischen demyelisierenden motorischen Neuropathie beim Säugetier, wobei das IFN-β intramuskulär zu verabreichen ist.

2. Verwendung gemäß Anspruch 1, wobei die chronische demyelisierende motorische Neuropathie chronische inflammatorische Demyelisierende Polyneuropathie (CIDP) ist.

3. Verwendung gemäß Anspruch 1 oder Anspruch 2, wobei das IFN-β reifes IFN-β umfasst.

4. Verwendung gemäß irgendeinem der Ansprüche 1 bis 3, wobei das IFN-β reifes IFN-β, welchem das erste Methionin fehlt, ist.

5. Verwendung gemäß irgendeinem der Ansprüche 1 bis 4, wobei das IFN-β humanes IFN-β ist.

6. Verwendung gemäß Anspruch 1, wobei das IFN-β zu mindestens 95 % identisch zu einem reifen menschlichen IFN-β in voller Länge, welches durch die SEQ ID NO:4 dargestellt wird, ist.

7. Verwendung gemäß Anspruch 6, wobei das IFN-β SEQ ID NO:4 umfasst.

8. Verwendung gemäß irgendeinem der Ansprüche 1 bis 7, wobei das IFN-β glykosyliert ist.

9. Verwendung gemäß irgendeinem der Ansprüche 1 bis 7, wobei das IFN-β nicht glykosyliert ist.

10. Verwendung gemäß Anspruch 1, wobei das IFN-β IFN-β-1a ist.

11. Verwendung gemäß Anspruch 1, wobei das IFN-β IFN-β-1b ist.

12. Verwendung gemäß irgendeinem der Ansprüche 1 bis 11, wobei das IFN-β mit der konstanten Domäne eines Immunoglobulin-Moleküls fusioniert ist.

13. Verwendung gemäß Anspruch 12, wobei das Immunoglobulin-Molekül ein humanes Immunoglobulin-Molekül ist.

14. Verwendung gemäß Anspruch 13, wobei das Immunoglobulin-Molekül die schwere Kette des IgG1 ist.

15. Verwendung gemäß Anspruch 14, wobei die Fusion des IFN-β mit der konstanten Domäne eines Immunoglobulin-Moleküls SEQ ID NO: 14 umfasst.

16. Verwendung gemäß irgendeinem der Ansprüche 1 bis 15, wobei das IFN-β ein pegyliertes IFN-β umfasst.

17. Verwendung gemäß irgendeinem der Ansprüche 1 bis 16, wobei das Arzneimittel ein stabilisierendes Agens umfasst.

18. Verwendung gemäß Anspruch 17, wobei das stabilisierende Agens eine saure Aminosäure ist.

19. Verwendung gemäß Anspruch 18, wobei das stabilisierende Agens Arginin ist.

20. Verwendung nach irgendeinem der Ansprüche 1 bis 19, wobei das Arzneimittel einen pH-Wert zwischen 4,0 und 7,2 hat.

21. Verwendung gemäß irgendeinem der Ansprüche 1 bis 20, wobei die Behandlung die Verabreichung von IFN-β an das Säugetier in Abständen von jeweils 1 bis 14 Tagen umfasst.

22. Verwendung gemäß Anspruch 21, wobei das IFN-β wöchentlich in einer Dosierung von 6 MIU zu verabreichen ist.

23. Verwendung gemäß Anspruch 21, wobei das IFN-β zweimal die Woche in einer Dosierung von 6 MIU zu verabreichen ist.

24. Verwendung gemäß Anspruch 21, wobei das IFN-β wöchentlich in einer Dosierung von 12 MIU zu verabreichen ist.

25. Verwendung gemäß Anspruch 21, wobei das IFN-β zweimal die Woche in einer Dosierung von 12 MIU zu verabreichen ist.

26. Verwendung gemäß irgendeinem der Ansprüche 1 bis 25, wobei das Säugetier ein Mensch ist.

27. Verwendung nach irgendeinem der Ansprüche 1 bis 26, wobei das Arzneimittel einer Versuchsperson, bei der zuvor keine Resistenz gegenüber anderen Behandlungen für chronische demyelisierende Neuropathie festgestellt wurde, zu verabreichen ist.

28. Verwendung gemäß irgendeinem der Ansprüche 1 bis 27, wobei das Arzneimittel innerhalb einer Kombinationsbehandlung, umfassend ein Immunsuppressivum oder Plasmapherese, zu verabreichen ist.

29. Verwendung gemäß Anspruch 28, wobei das Arzneimittel in einer Kombinationsbehandlung, welche ein Immunsuppressivum ausgewählt aus der Gruppe bestehend aus einem Steroid, Azothioprin, Cyclosporin, Cyclophosphamid, und Mycophenolat, umfasst, zu verabreichen ist.

30. Verwendung gemäß irgendeinem der Ansprüche 1 bis 27, wobei die chronische demyelisierende motorische Neuropathie eine chronische inflammatorische Demyelisierende Polyneuropathie (CIDP) ist, und wobei das Arzneimittel in einer Kombinationsbehandlung umfassend eine zweite CIDP-Behandlung ausgewählt aus der Gruppe bestehend aus der Verabreichung von IVIg; Verabreichung eines Steroids; Verabreichung einer entzündungshemmenden Arznei und Plasmaphrerese, zu verabreichen ist.

31. Verwendung gemäß Anspruch 30, wobei das IFP-β wöchentlich zu verabreichen ist.

32. Verwendung gemäß irgendeinem der Ansprüche 1 bis 27, wobei das Arzneimittel an eine Versuchsperson, welche mit einer anderen Behandlung für eine chronische demyelisierende motorische Neuropathie behandelt wurde, ausgewählt aus der Gruppe bestehend aus der Verabreichung von IVIg; Verabreichung eines Steroids; Verabreichung einer entzündungshemmenden Arznei und Plasmaphrerese, zu verabreichen ist.

33. Ein IFN-β, zur Verwendung bei der Behandlung einer chronischen demyelisierenden motorischen Neuropathie beim Säugetier, wobei das IFN-β intramuskulär verabreicht wird.

34. Ein IFN-β, zur Verwendung bei einer Methode zur Behandlung von CIDP, wobei die Methode die intramuskuläre Verabreichung einer pharmazeutisch wirksamen Menge von IFN-β an eine Versuchsperson, welche an CIDP erkrankt ist, und weiterhin die Verabreichung eines Immunsuppresivum an die Versuchsperson oder das Unterziehen der Versuchsperson der Plasmapherese, umfasst.

35. IFN-β zur Verwendung bei einer Methode zur Behandlung von CIDP gemäß Anspruch 34, wobei die Methode zur Behandlung von CIDP die Verabreichung eines Immunsupressivums ausgewählt aus der Gruppe bestehend aus einem Steroid, Azothioprin, Cyclosporin, Cyclophosphamid, und Mycophenolat, umfasst.

36. Ein IFP-β, zur Verwendung bei einer Methode zur Behandlung von CIDP, wobei diese Methode die Verabreichung einer pharmazeutisch effektiven Menge eines IFP in Kombination mit einer zweiten CDIP Behandlung, ausgewählt aus der Gruppe bestehend aus Verabreichung von IVIg; Verabreichung eines Steroids; Verabreichung einer entzündungshemmenden Arznei und Plasmapherese, und wobei die Verabreichung des IFN-β intramuskulär ist, umfasst.

37. IFP-β zur Verwendung bei einer Methode zur Behandlung von CIDP gemäß Anspruch 36, wobei die Verabreichung des IFP-β wöchentlich erfolgt.

## Revendications

1. Utilisation d'un INFβ dans la production d'un médicament destiné au traitement d'un neuropathie motrice démyélinisante chronique chez un mammifère, dans laquelle l'INFβ doit être administré par voie intramusculaire.

2. Utilisation selon la revendication 1, dans laquelle la neuropathie motrice démyélinisante chronique est la neuropathie démyélinisante inflammatoire chronique (CIDP).

3. Utilisation selon la revendication 1 ou la revendication 2, dans laquelle l'INFβ comprend le INFβ mature.

4. Utilisation selon l'une quelconque des revendications 1 à 3, dans laquelle l'INFβ est l'INFβ mature qui est dépourvu de la première méthionine.

5. Utilisation selon l'une quelconque des revendications 1 à 4, dans laquelle l'INFβ est l'INFβ humain.

6. Utilisation selon la revendication 1, dans laquelle l'INFβ est au moins identique à 95 % à l'INFβ humain mature complet ayant SEQ ID N° : 4.

7. Utilisation selon la revendication 6, dans laquelle l' INFβ comprend SEQ ID N° : 4.

8. Utilisation selon l'une quelconque des revendications 1 à 7, dans laquelle l'INFβ est glycosylé.

9. Utilisation selon l'une quelconque des revendications 1 à 7, dans laquelle l'INFβ n'est pas glycosylé.

10. Utilisation selon la revendication 1, dans laquelle l'INFβ est l'INFβ-1a.

11. Utilisation selon la revendication 1, dans laquelle l'INFβ est l' l'INFβ-1b.

12. Utilisation selon l'une quelconque des revendications 1 à 11, dans laquelle l'INFβ est fusionné au domaine constant d'une molécule d'immunoglobuline.

13. Utilisation selon la revendication 12, dans laquelle la molécule d'immunoglobuline est une molécule d'immunoglobuline humaine.

14. Utilisation selon la revendication 13, dans laquelle la molécule d'immunoglobuline est la chaîne lourde d'IgG1.

15. Utilisation selon la revendication 14, dans laquelle la fusion de l'INFβ au domaine constant d'une molécule d'immunoglobuline comprend SEQ ID N° : 14.

16. Utilisation selon l'une quelconque des revendications 1 à 15, dans laquelle l'INFβ comprend un INFβ pégylé.

17. Utilisation selon l'une quelconque des revendications 1 à 16, dans laquelle le médicament comprend un agent stabilisant.

18. Utilisation selon la revendication 17, dans laquelle l'agent stabilisant est un acide aminé acide.

19. Utilisation selon la revendication 18, dans laquelle l'agent stabilisant est l'arginine.

20. Utilisation selon l'une quelconque des revendications 1 à 19, dans laquelle le médicament a un pH entre 4,0 et 7,2.

21. Utilisation selon l'une quelconque des revendications 1 à 20, dans laquelle le traitement comprend l'administration au mammifère d'un INFβ à intervalle de 1 à 14 jours.

22. Utilisation selon la revendication 21, dans laquelle l'INFβ doit être administrée de façon hebdomadaire à une dose de 6 MUI.

23. Utilisation selon la revendication 21, dans laquelle l'INFβ doit être administré deux fois par semaines à une dose de 6 MUI.

24. Utilisation selon la revendication 21, dans laquelle l'INFβ doit être administré de façon hebdomadaire à une dose de 12 MUI.

25. Utilisation selon la revendication 21, dans laquelle l'INFβ doit être administré deux fois par semaine à une dose de 12 MUI.

26. Utilisation selon l'une quelconque des revendications 1 à 25, dans laquelle le mammifère est un être humain.

27. Utilisation selon l'une quelconque des revendications 1 à 26, dans laquelle le médicament doit être administré à un sujet chez lequel on n'a pas constaté précédemment qu'il était résistant à d'autres traitements de la neuropathie démyélinisante chronique.

28. Utilisation selon l'une quelconque des revendications 1 à 27, dans laquelle le médicament doit être administré dans un traitement combiné comprenant un immunosuppresseur ou une plasmaphérèse.

29. Utilisation selon la revendication 28, dans laquelle le médicament doit être administré dans un traitement combiné comprenant un immunosuppresseur choisi dans le groupe constitué d'un stéroïde, de l'azothioprine, de la cyclosporine, du cyclophosphamide et du mycophénolate.

30. Utilisation selon l'une quelconque des revendications 1 à 27, dans laquelle la neuropathie motrice démyélinisante chronique est la neuropathie démyélinisante inflammatoire chronique (CIDP) et dans laquelle le médicament doit être administré dans un traitement combiné comprenant un deuxième traitement de la CIDP choisi dans le groupe constitué de l'administration d'IVIg, de l'administration d'un stéroïde, de l'administration d'un médicament anti-inflammatoire et d'une plasmaphérèse.

31. Utilisation selon la revendication 30, dans laquelle l'INFβ doit être administré de façon hebdomadaire.

32. Utilisation selon l'une quelconque des revendications 1 à 27, dans laquelle le médicament doit être administré à un sujet qui est traité par un autre traitement d'une neuropathie motrice démyélinisante chronique choisi dans le groupe constitué de l'administration d'IVIg, de l'administration d'un stéroïde, de l'administration d'un médicament anti-inflammatoire et d'une plasmaphérèse et le traitement comprend en outre l'élimination progressive de l'autre traitement.

33. INFβ pour utilisation dans le traitement d'une neuropathie motrice démyélinisante chronique chez un mammifère, où l'INFβ est administré par voie intramusculaire.

34. INFβ, pour utilisation dans un procédé de traitement de la CIDP, où le procédé comprend l'administration à un sujet souffrant de CIDP, d'une quantité pharmaceutiquement efficace d'un INFβ par une voie intramusculaire et en outre l'administration au sujet d'un immunosuppresseur ou la soumission du sujet à une plasmaphérèse.

35. INFβ, pour utilisation dans un procédé de traitement de la CIDP selon la revendication 34, où le procédé de traitement de la CIDP comprend l'administration au sujet d'un immunosuppresseur choisi dans le groupe constitué d'un stéroïde, de l'azothioprine, de la cyclosporine, du cyclophosphamide et du mycophénolate.

36. INFβ, pour utilisation dans un procédé de traitement de la CIDP, où le procédé comprend l'administration au sujet souffrant de la CIDP d'une quantité pharmaceutiquement efficace d'un INFβ en combinaison avec un deuxième traitement de la CIDP choisi dans le groupe constitué de l'administration d'IVIg, de l'administration d'un stéroïde, de l'administration d'un médicament anti-inflammatoire et d'une plasmaphérèse et où l'administration de l'INFβ s'effectue par voie intramusculaire.

37. INFβ, pour utilisation dans un procédé de traitement de la CIDP selon la revendication 36, où l'administration de l'INFβ est hebdomadaire.
